# EUROPEAN PATENT APPLICATION

(11) **EP 4 678 746 A1**
(43) Date of publication of application: **14.01.2026**
(21) Application number: 24769867.3
(22) Date of filing: 08.03.2024
(51) Int. Cl.: C12N 15/113, A61K 31/713, A61K 48/00, A61P 3/06

(54) **PHARMACEUTICAL COMPOSITION AND USE THEREOF**

(30) Priority: 10.03.2023 CN 202310231935
(71) Applicant: Suzhou Ribo Life Science Co., Ltd., Suzhou, Jiangsu 215300 (CN)
(72) Inventor: GAN, Liming, Suzhou, Jiangsu 215300 (CN); GAO, Shan, Suzhou, Jiangsu 215300 (CN); LIANG, Zicai, Suzhou, Jiangsu 215300 (CN); ZHANG, Hongyan, Suzhou, Jiangsu 215300 (CN)
(74) Representative: Kraus & Lederer PartGmbB
(86) International application number: PCT/CN2024/080732
(87) International publication number: WO 2024/188174

(57) **Abstract**

The present disclosure provides a pharmaceutical composition, comprising a first RNAi agent and a second RNAi agent, the first RNAi agent comprises one or more first siRNAs capable of inhibiting APOC3 mRNA and/or one or more first siRNA conjugates; the second RNAi agent comprises one or more second siRNAs capable of inhibiting PCSK9 mRNA and/or one or more second siRNA conjugates; and on the basis of a total amount of siRNAs and siRNA groups, a weight ratio of the first RNAi agent to the second RNAi agent in the pharmaceutical composition is 10: 1 to 1: 10. The pharmaceutical composition and a treatment method can be used for effectively treating and/or preventing a disease or symptom related to hyperlipidemia.

## Description

### TECHNICAL FIELD

The present disclosure relates to a pharmaceutical composition capable of treating and/or preventing a disease or symptom related to hyperlipidemia. The present disclosure further relates to use of the pharmaceutical composition and a method for treating and/or preventing a disease or symptom related to hyperlipidemia.

### BACKGROUND

Dyslipidemia, also known as hyperlipidemia, is a systemic disease with abnormal lipid metabolism or operation, which makes plasma lipid higher than a normal value, wherein persistently high low-density lipoprotein cholesterol (LDL-c) is an important risk factor for inducing and promoting the occurrence and development of atherosclerosis, which is closely related to ischemic cardiovascular and cerebrovascular diseases, so that the disease is seriously threatening the health of patients all over the world.

Studies have shown that apolipoprotein C3 (*APOC3*) plays an important role in lipid metabolism, an expression quantity of *APOC3* in blood circulation of a person with an *APOC3* mutant gene is decreased by 46%, and a level of triglyceride in plasma is decreased by 39% compared with that of an ordinary person. A proprotein convertase subtilisin/kexin type 9 *(PCSK9)* gene plays an important role in lipid metabolism, especially cholesterol metabolism, and a level of total cholesterol (TC) in plasma can be effectively reduced by inhibiting the expression of *PCSK9* gene and/or *APOC3* gene.

Therefore, silencing gene expression at a gene level by using small interfering RNA (siRNA) to block the *APOC3* and the *PCSK9* is undoubtedly an ideal means to treat dyslipidemia. However, current studies have shown that it is difficult to reduce the LDL-c to a very low level by separately administering the siRNA for inhibiting the *APOC3* or the *PCSK9* even if an administration dosage of the siRNA is continuously increased.

### SUMMARY

The inventors of the present disclosure have unexpectedly found that combined use of an RNAi agent capable of inhibiting *APOC3* mRNA and an RNAi agent capable of inhibiting *PCSK9* mRNA in a specific proportion or dosage in preventing and/or treating a disease or symptom related to hyperlipidemia shows a high synergistic effect, which can not only reduce a level of TC in plasma, but also stably and significantly reduce a level of LDL-c in plasma for a long time, thereby showing an excellent therapeutic effect.

In one aspect, the present disclosure provides a pharmaceutical composition, the pharmaceutical composition comprises a first RNAi agent and a second RNAi agent, wherein the first RNAi agent comprises one or more first siRNAs and/or one or more first siRNA conjugates, each first siRNA is independently a siRNA capable of inhibiting *APOC3* mRNA, and each first siRNA conjugate comprises a siRNA group formed by one first siRNA and a conjugating group conjugated with the siRNA;
the second RNAi agent comprises one or more second siRNAs and/or one or more second siRNA conjugates, each second siRNA is independently a siRNA capable of inhibiting *PCSK9* mRNA, and each second siRNA conjugate comprises a siRNA group formed by one second siRNA and a conjugating group conjugated with the siRNA; and
on the basis of a total amount of siRNAs and siRNA groups in the first RNAi agent and the second RNAi agent respectively, a weight ratio of the first RNAi agent to the second RNAi agent in the pharmaceutical composition is 10: 1 to 1: 10.

In another aspect, the present disclosure further provides use of the pharmaceutical composition in preparation of a drug for treating a disease or symptom related to hyperlipidemia.

In another aspect, the present disclosure further provides a method for treating and/or perventing a disease or symptom related to hyperlipidemia, the method comprises administering a first RNAi agent and a second RNAi agent to a subject in need, wherein the first RNAi agent comprises one or more first siRNAs and/or one or more first siRNA conjugates, each first siRNA is independently a siRNA capable of inhibiting *APOC3* mRNA, and each first siRNA conjugate comprises a siRNA group formed by one first siRNA and a conjugating group conjugated with the siRNA;
the second RNAi agent comprises one or more second siRNAs and/or one or more second siRNA conjugates, each second siRNA is a siRNA capable of inhibiting *PCSK9* mRNA, and each second siRNA conjugate comprises a siRNA group formed by one second siRNA and a conjugating group conjugated with the siRNA; and
on the basis of a total amount of siRNAs and siRNA groups in the first RNAi agent and the second RNAi agent respectively, a weight ratio of the first RNAi agent to the second RNAi agent administered to the subject is 10: 1 to 1: 10.

### Incorporation by reference

All publications, patents and patent applications mentioned in the specification are incorporated herein by reference to the same extent as if each separate publication, patent or patent application is specifically and separately incorporated herein by reference.

### Advantageous Effects

Compared with separate administration of the RNAi agent for inhibiting the *APOC3* mRNA or the RNAi agent for inhibiting the *PCSK9* mRNA, the composition and the treatment method provided by the present disclosure both have a significantly better effect on reducing the level of TC and the level of LDL-c, show a significant synergistic effect, especially on reducing the level of LDL-c which has attracted much attention, and unexpectedly break through a limitation on reducing the level of LDL-c by separate administration of the first RNAi agent or the second RNAi agent. The pharmaceutical composition and the treatment method of the present disclosure have a good application prospect in preventing and/or treating a symptom or disease related to hyperlipidemia.

For a change of the level of TC, on one hand, under the condition that the total administration dosages are all 6 mg/Kg, mice separately administered with 6 mg/Kg RNAi agent for inhibiting the *PCSK9* mRNA show almost no reduction of TC *in vivo,* while mice administered with the pharmaceutical composition of the present disclosure (3 mg/Kg RNAi agent for inhibiting the *APOC3* mRNA and 3 mg/Kg RNAi agent for inhibiting the *PCSK9* mRNA) show more than 45% reduction rate of TC *in vivo* (reduction rate of TC = 100% - normalized value of content of TC); even if reduction rates of TC in mice in the groups administered with the RNAi agent for inhibiting the *APOC3* mRNA and the RNAi agent for inhibiting the *PCSK9* mRNA respectively are added, a sum of the reduction rates of TC is also lower than 45%. Under the condition that the total administration dosages are all 4 mg/Kg, when reduction rates of TC in mice in the groups administered with 1 mg/Kg RNAi agent for inhibiting the *APOC3* mRNA and 3 mg/Kg RNAi agent for inhibiting the *PCSK9* mRNA respectively are added, a maximum sum of the reduction rates of TC is 34.9%, while a reduction rate of TC in mice administered with the pharmaceutical composition of the present disclosure (1 mg/Kg RNAi agent for inhibiting the *APOC3* mRNA and 3 mg/Kg RNAi agent for inhibiting the *PCSK9* mRNA) can reach 37.2%. On the other hand, taking a change of a level of TC on an 8^{th} day after administration as an example, under the condition that the total administration dosages are all 6 mg/Kg, mice in the group separately administered with 6 mg/Kg RNAi agent for inhibiting the *PCSK9* mRNA show no reduction of TC *in vivo,* while mice in the group administered with the pharmaceutical composition of the present disclosure (3 mg/Kg RNAi agent for inhibiting the *APOC3* mRNA and 3 mg/Kg RNAi agent for inhibiting the *PCSK9* mRNA) show 45.6% reduction rate of TC. Even if reduction rates of TC in mice in the groups administered with the RNAi agent for inhibiting the *APOC3* mRNA (3 mg/Kg) and the RNAi agent for inhibiting the *PCSK9* mRNA (3mg/Kg) respectively are added, a sum of the reduction rates of TC is only 33.3%. Under the condition that the total administration dosages are all 4 mg/Kg, when reduction rates of TC in mice in the groups administered with 1 mg/Kg RNAi agent for inhibiting the *APOC3* mRNA and 3 mg/Kg RNAi agent for inhibiting the *PCSK9* mRNA respectively are added, a maximum sum of the reduction rates of TC is 34.9%, while a reduction rate of TC in mice in the group administered with the pharmaceutical composition of the present disclosure (1 mg/Kg RNAi agent for inhibiting the *APOC3* mRNA and 3 mg/Kg RNAi agent for inhibiting the *PCSK9* mRNA) can reach 37.0%.

For a change of the level of LDL-c, on one hand, under the condition that the total administration dosages are all 6 mg/Kg, mice separately administered with the RNAi agent for inhibiting the *PCSK9* mRNA show 43.3% maximum reduction rate of LDL-c *in vivo* (reduction rate of LDL-c = 100% - normalized value of content of LDL-c), while mice administered with the pharmaceutical composition of the present disclosure show a reduction rate of LDL-c as high as 72.8% *in vivo.* Moreover, the level of LDL-c in plasma cannot be reduced to below 50% regardless of the separate administration of the high-dose RNAi agent for inhibiting the *APOC3* mRNA or the separate administration of the high-dose RNAi agent for inhibiting the *PCSK9* mRNA, but the mice administered with the low-dose (4 mg/kg) pharmaceutical composition of the present disclosure have already shown a reduction rate of LDL-c as high as 67% *in vivo.* Taking a change of a level of LDL-c on an 8^{th} day after administration as an example, under the condition that the total administration dosages are all 6 mg/Kg, mice in the group separately administered with the RNAi agent for inhibiting the *PCSK9* mRNA show 41.4% reduction rate of LDL-c, while mice in the group administered with the pharmaceutical composition of the present disclosure (3 mg/Kg RNAi agent for inhibiting the *APOC3* mRNA and 3 mg/Kg RNAi agent for inhibiting the *PCSK9* mRNA) show a reduction rate of LDL-c as high as 72.8%.

### EMBODIMENT

The specific embodiments of the present disclosure are described in detail as below. It should be understood that the specific embodiments described herein are only for the purpose of illustration and explanation of the present disclosure and are not intended to limit the present disclosure.

In the present disclosure, unless otherwise stated, *APOC* mRNA or *"POC3* gene-expressed mRNA" refers to the mRNA with the sequence shown in Genbank registration number NM_000040.3, and the *APOC3* gene refers to a gene capable of transcribing the above *APOC3* mRNA. *PCSK9* mRNA or *"PCSK9* gene-expressedm RNA" refers to the mRNA with the sequence shown in Genbank registration number NM_174936.3, and the *PCSK9* gene refers to a gene capable of transcribing the above *PCSK9* mRNA. *ANGPTL3* mRNA or *"ANGPTL3* gene-expressed mRNA" refers to the mRNA with the sequence shown in Genbank registration number NM_014495.4, and the *ANGPTL3* gene refers to a gene capable of transcribing the above *ANGPTL3* mRNA.

### Definition

In the context of the present disclosure, unless otherwise specified, capital letters C, G, U, and A indicate the base composition of the nucleotides; the lowercase m indicates that the nucleotide adjacent to the left side of the letter m is a methoxy modified nucleotide; the lowercase f indicates that the nucleotide adjacent to the left side of the letter f is a fluoro modified nucleotide; the lowercase letter s indicates that the two nucleotides adjacent to the left and right of the letter s are linked by phosphorothioate; P1 represents that the nucleotide adjacent to the right side of P1 is a 5'-phosphate nucleotide or a 5'-phosphate analogue modified nucleotide, in some embodiments, P1 represents specifically modified VP, Ps or P, wherein the letter combination VP represents that the nucleotide adjacent to the right side of the letter combination VP is a 5'-(E)-vinylphosphonate (E-VP) modified nucleotide, the letter combination Ps represents that the nucleotide adjacent to the right side of the letter combination Ps is a phosphorothioate modified nucleotide, the capital letter P represents that the nucleotide adjacent to the right side of the letter P is a 5'-phosphate nucleotide, and the underlined capital letter S indicates that the nucleotide adjacent to the left side of the letter S is a stably modified nucleotide. Each U base and T base in all sequences of the present disclosure can be interchangeably used, which will not significantly affect the activity or off-target effect of siRNA of the present disclosure.

In the context of the present disclosure, the "fluoro modified nucleotide" refers to a nucleotide formed by substituting the 2'-hydroxy of the ribose group of the nucleotide with fluoro, and the "non-fluoro modified nucleotide" refers to a nucleotide formed by substituting the 2'-hydroxy of the ribose group of the nucleotide with a non-fluoro group, or a nucleotide analogue. These nucleotides formed by substituting the 2'-hydroxy of the ribose group with the non-fluorine group are well known to those skilled in the art, and these nucleotides may be selected from one of a 2'-alkoxy modified nucleotide, a 2'-alkyl modified nucleotide, a 2'-substituted alkyl modified nucleotide, a 2'-amino modified nucleotide, a 2'-substituted amino modified nucleotide and a 2'-deoxy nucleotide. In some embodiments, the 2'-alkoxy modified nucleotide may be methoxy modified nucleotide. The "methoxy modified nucleotide" refers to a nucleotide formed by substituting the 2'-hydroxy of the ribose group with a methoxy group. The "nucleotide analogue" refers to a group that can replace a nucleotide in a nucleic acid, while structurally differs from an adenine ribonucleotide, a guanine ribonucleotide, a cytosine ribonucleotide, a uracil ribonucleotide or a thymidine deoxyribonucleotide, such as an isonucleotide, a bridged nucleic acid (BNA) or an acyclic nucleotide.

In some embodiments, the "non-fluoro modified nucleotide" may also be a stably modified nucleotide, the stably modified nucleotide refers to a nucleotide formed by substituting the 2'-hydroxyl of the ribose group of the nucleotide with a stably modified group, and compared with a siRNA with an unmodified nucleotide at a corresponding site, a siRNA with the stably modified nucleotide has improved thermal stability. The stably modified nucleotide refers to a nucleotide formed by substituting the 2'-hydroxy of the ribose group of the nucleotide with a stably modified group, and compared with a siRNA with an unmodified nucleotide at a corresponding site, a siRNA with the stably modified nucleotide has improved thermal stability. In some embodiments, the stably modified group is selected from one or more of 2'-O-methoxyethyl, 2'-O-allyl, 2'-C-allyl, 2'-O-2-N-methylamino-2-oxyethylene, 2'-O-2-N,N-dimethylaminoethyl, 2'-O-3-aminopropyl and 2'-O-2,4-dinitrophenyl.

In the context of the present disclosure, expressions "complementary" and "reverse complementary" can be interchangeably used, and have a well-known meaning in the art, namely, the bases in one strand are complementarily paired with those in the other strand of a double-stranded nucleic acid molecule. In DNA, a purine base adenine (A) is always paired with a pyrimidine base thymine (T) (or uracil (U) in RNAs); and a purine base guanine (G) is always paired with a pyrimidine base cytosine (C). Each base pair comprises a purine and a pyrimidine. While adenines in one strand are always paired with thymines (or uracils) in another strand, and guanines are always paired with cytosines, these two strands are considered as being complementary to each other; and the sequence of a strand may be deduced from the sequence of its complementary strand. Correspondingly, a "mispairing" in the art means that in a double-stranded nucleic acid, the bases at corresponding sites are not presented in a manner of being complementarily paired.

In the context of the present disclosure, unless otherwise specified, "basically reverse complementary" means that there are no more than 3 base mispairings between two nucleotide sequences. "Substantially reverse complementary" means that there is no more than 1 base mispairing between two nucleotide sequences. "Completely complementary" means that there is no based mispairing between two nucleotide sequences.

In the context of the present disclosure, particularly in the description of the method for preparing the siRNA, the pharmaceutical composition or the siRNA conjugate of the present disclosure, unless otherwise specified, the nucleoside monomer refers to, according to the kind and sequence of the nucleotides in the siRNA or siRNA conjugate to be prepared, unmodified or modified RNA phosphoramidites used in a solid phase phosphoramidite synthesis (the RNA phosphoramidites are also called as Nucleoside phosphoramidites elsewhere). Solid phase phosphoramidite synthesis is a well-known method used in RNA synthesis to those skilled in the art. Nucleoside monomers used in the present disclosure can all be commercially available.

Those skilled in the art would understand, with respect to any group containing one or more substituents, that such groups are not intended to introduce any substitution or substitution patterns that are sterically impractical, synthetically infeasible and/or inherently unstable.

As used herein, "alkyl" refers to straight chain and branched chain having the indicated number of carbon atoms, usually 1 to 20 carbon atoms, for example 1 to 10 carbon atoms, such as 1 to 8 or 1 to 6 carbon atoms. For example, C₁-C₆ alkyl encompasses both straight and branched chain alkyl of 1 to 6 carbon atoms. When naming an alkyl residue having a specific number of carbon atoms, all branched and straight chain forms having that number of carbon atoms are intended to be encompassed. Thus, for example, "butyl" is meant to include n-butyl, sec-butyl, isobutyl and t-butyl; and "propyl" includes n-propyl and isopropyl. Alkylene is a subset of alkyl, referring to the same residues as alkyl, but having two attachment positions.

As used herein, "alkenyl" refers to an unsaturated branched or linear alkyl having at least one carbon-carbon double bond which is obtained by respectively removing one hydrogen molecule from two adjacent carbon atoms of the parent alkyl. The group may be in either cis or trans configuration of the double bond. Typical alkenyl groups include, but not limited to, ethenyl; propenyls such as prop-1-en-1-yl, prop-1-en-2-yl, prop-2-en-1-yl (allyl), and prop-2-en-2-yl; and butenyls such as but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl, and the like. In certain embodiments, an alkenyl group has 2 to 20 carbon atoms, and in other embodiments, 2 to 10, 2 to 8, or 2 to 6 carbon atoms. Alkenylene is a subset of alkenyl, referring to the same residues as alkenyl, but having two attachment positions.

As used herein, "alkynyl" refers to an unsaturated branched or linear alkyl having at least one carbon-carbon triple bond which is obtained by respectively removing two hydrogen molecules from two adjacent carbon atoms of the parent alkyl. Typical alkynyl groups include, but not limited to, ethynyl; propynyls such as prop-1-yn-1-yl, and prop-2-yn-1-yl; and butynyls such as but-1-yn-1-yl, but-1-yn-3-yl, but-3-yn-1-yl, and the like. In certain embodiments, an alkynyl group has 2 to 20 carbon atoms, and in other embodiments, 2 to 10, 2 to 8, or 2 to 6 carbon atoms. Alkynylene is a subset of alkynyl, referring to the same residues as alkynyl, but having two attachment positions.

As used herein, "alkoxy" refers to an alkyl group of the indicated number of carbon atoms attached through an oxygen bridge, such as, methoxy, ethoxy, propoxy, isopropoxy, n-butoxy, sec-butoxy, tert-butoxy, pentyloxy, 2-pentyloxy, isopentyloxy, neopentyloxy, hexyloxy, 2-hexyloxy, 3-hexyloxy, 3-methylpentyloxy, and the like. An alkoxy usually has 1 to 10, 1 to 8, 1 to 6, or 1 to 4 carbon atoms attached through oxygen bridge.

As used herein, "aryl" refers to a group derived from an aromatic monocyclic or multicyclic hydrocarbon ring system by removing a hydrogen atom from a ring carbon atom. The aromatic monocyclic or multicyclic hydrocarbon ring system contains only hydrogen and 6 to 18 carbon atoms, wherein at least one ring in the ring system is fully unsaturated, i.e., containing a cyclic, delocalized (4n+2) π-electron system in accordance with the Hückel theory. Aryl groups include, but not limited to, phenyl, fluorenyl, naphthyl and the like. Arylene is a subset of aryl, referring to the same residues as aryl, but having two attachment positions.

"Heteroaryl" refers to a group derived from a 5- to 18-membered aromatic ring radical that comprises 2 to 17 carbon atoms and 1 to 6 heteroatoms selected from nitrogen, oxygen and sulfur. As used herein, the heteroaryl may be a monocyclic, bicyclic, tricyclic or tetracyclic ring system, wherein at least one ring in the ring system is fully unsaturated, i.e., containing a cyclic, delocalized (4n+2) π-electron system in accordance with the Hückel theory. The heteroaryl includes fused or bridged ring systems. In some embodiments, the heteroatoms in the heteroaryl are oxidized heteroatoms. In some embodiments, the heteroaryl includes one or more nitrogen atoms. In some embodiments, one or more of the nitrogen atoms in the heteroaryl are quaternized nitrogen atoms. The heteroaryl may be linked to the rest of the molecule through any atom of the ring. Examples of such heteroaryl include, but not limited to, azepinyl, acridinyl, benzimidazolyl, benzindolyl, 1,3-benzodioxazolyl, benzofuranyl, benzoxazolyl, benzo[d]thiazolyl, benzothiadiazolyl, benzo[b][1,4]dioxepinyl, benzo[b][1,4]oxazinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl, benzothieno[3,2-d]pyrimidinyl, benzotriazolyl, benzo[4,6]imidazo[1,2-a]pyridinyl, carbazolyl, cinnolinyl, cyclopenta[d]pyrimidinyl, 6,7-dihydro-5H-cyclopenta[4,5]thieno[2,3-d]pyrimidinyl, 5,6-dihydrobenzo[h]quinazolinyl, 5,6-dihydrobenzo[h]cinnolinyl, 6,7-dihydro-5H-benzo[6,7]cyclohepta[1,2-c]pyridazinyl, dibenzofuranyl, dibenzothienyl, furanyl, furanonyl, furo[3,2-c]pyridinyl, 5,6,7,8,9,10-hexahydrocycloocta[d] pyrimidinyl, 5,6,7,8,9,10-hexahydrocycloocta[d]pyridazinyl, 5,6,7,8,9,10-hexahydrocycloocta[d]pyridinyl, isothiazolyl, imidazolyl, indazolyl, indolyl, isoindolyl, indolinyl, isoindolinyl, isoquinolyl, indolizinyl, isoxazolyl, 5,8-methano-5,6,7,8-tetrahydroquinazolinyl, naphthyridinyl, 1,6-naphthyridinonyl, oxadiazolyl, 2-oxoazepinyl, oxazolyl, oxiranyl, 5,6,6a,7,8,9,10,10a-octahydrobenzo[H]quinazolinyl, 1-phenyl-1H-pyrrolyl, phenazinyl, phenothiazinyl, phenoxazinyl, phthalazinyl, pteridinyl, purinyl, pyrrolyl, pyrazolyl, pyrazolo[3,4-d]pyrimidinyl, pyridinyl, pyrido[3,2-d]pyrimidinyl, pyrido[3,4-d]pyrimidinyl, pyrazinyl, pyrimidinyl, pyridazinyl, quinazolinyl, quinoxalinyl, quinolinyl, tetrahydroquinolinyl, 5,6,7,8-tetrahydroquinazolinyl, 5,6,7,8-tetrahydrobenzo[4,5]thieno[2,3-d]pyrimidinyl, 6,7,8,9-tetrahydro-5H-cyclohepta[4,5]thieno[2,3-d]pyrimidinyl, 5,6,7,8-tetrahydropyrido[4,5-c]pyridazinyl, thiazolyl, thiadiazolyl, triazolyl, tetrazolyl, triazinyl, thieno[2,3-d]pyrimidinyl, thieno[3,2-d]pyrimidinyl, thieno[2,3-c]pridinyl, and thiophenyl/thienyl.

Various hydroxy protecting groups may be used in the present disclosure. In general, protecting groups render chemical functional groups inert to specific reaction conditions, and may be attached to and removed from such functional groups in a molecule without substantially damaging the remainder of the molecule. Representative hydroxy protecting groups are disclosed in Beaucage, et al., Tetrahedron 1992, 48, 2223-2311, and also in Greene and Wuts, Protective Groups in Organic Synthesis, Chapter 2, 2d ed, John Wiley & Sons, New York, 1991, each of which is hereby incorporated by reference in their entirety. In some embodiments, the protecting group is stable under basic conditions but can be removed under acidic conditions. In some embodiments, non-exclusive examples of the hydroxy protecting groups used herein include dimethoxytrityl (DMT), monomethoxytrityl, 9-phenylxanthen-9-yl (Pixyl), or 9-(p-methoxyphenyl)xanthen-9-yl (Mox). In some embodiments, non-exclusive examples of the hydroxy protecting groups used herein include Tr (trityl), MMTr (4-methoxytrityl), DMTr (4,4'-dimethoxytrityl), and TMTr (4,4',4"-trimethoxytrityl).

The term "subject", as used herein, refers to any animal, e.g., mammal or marsupial. The subject of the present disclosure includes, but not limited to, human, non-human primate (e.g., rhesus or other kinds of macaque), mouse, pig, horse, donkey, cow, rabbit, sheep, rat and any kind of poultry.

### Pharmaceutical composition of the present disclosure

In one aspect, the present disclosure provides a pharmaceutical composition for treating and/or preventing a disease or symptom related to hyperlipidemia. As used herein, "treatment" refers to a method for obtaining advantageous or desired result, including but not limited to, therapeutic benefit. "Therapeutic benefit" means eradication or improvement of potential disorder to be treated. Moreover, the therapeutic benefit is achieved by eradicating or ameliorating one or more of physiological symptoms associated with the potential disorder such that an improvement is observed in the subject, notwithstanding that the subject may still be afflicted with the potential disorder.

As used herein, "prevention" refers to a method for obtaining advantageous or desired result, including but not limited to, prophylactic benefit. For obtaining the "prophylactic benefit", the pharmaceutical composition of the present disclosure may be administered to the subject at risk of developing a particular disease, or to the subject reporting one or more physiological symptoms of a disease, even though the diagnosis of this disease may not have been made. The "preventing a disease or symptom related to hyperlipidemia" means that, when the subject begins to have a tendency of blood lipid increase, the blood lipid in the subject is prevented from increasing to an abnormal level by administering the pharmaceutical composition of the present disclosure to the subject, and the abnormal level refers to that a level of total cholesterol (TC) in the human subject exceeds 6.2 mmol/L, a level of triglyceride (TG) exceeds 2.3 mmol/L, and/or a level of low-density lipoprotein cholesterol (LDL) exceeds 4.1 mmol/L.

The pharmaceutical composition of the present disclosure comprises a first RNAi agent and a second RNAi agent, wherein the first RNAi agent comprises one or more first siRNAs and/or one or more first siRNA conjugates, each first siRNA is independently a siRNA capable of inhibiting *APOC3* mRNA, and each first siRNA conjugate comprises a siRNA group formed by one first siRNA and a conjugating group conjugated with the siRNA.

The second RNAi agent comprises one or more second siRNAs and/or one or more second siRNA conjugates, each second siRNA is independently a siRNA capable of inhibiting *PCSK9* mRNA, and each second siRNA conjugate comprises a siRNA group formed by one second siRNA and a conjugating group conjugated with the siRNA.

In the context of the present disclosure, unless otherwise stated, "conjugating" refers to two or more chemical moieties each with specific function being linked to each other via a covalent linkage. Correspondingly, a "conjugate" refers to a compound formed by covalent linkage of individual chemical moieties. Further, a "siRNA conjugate" represents a compound formed by covalently linking one or more chemical moieties with specific functions to siRNA. The siRNA conjugate should be understood according to the context as the generic term of a plurality of siRNA conjugates or siRNA conjugates shown in certain chemical formulae. In the context of the present disclosure, a "conjugating molecule" should be understood as a specific compound capable of being conjugated to a siRNA via reactions, thus finally forming the siRNA conjugate of the present disclosure. As used in the present disclosure, a "siRNA conjugate" comprises a siRNA group and a conjugating group part, wherein the siRNA group refers to a chemical moiety formed by removing one or more atoms from a siRNA molecule. It can be understood by those skilled in the art that the removal of one or more atoms above will not destroy the inhibitory activity or stability of the siRNA on a target mRNA. For example, the siRNA group may be a chemical moiety formed by removing the hydrogen atom in the phosphate bond, or a chemical moiety formed by removing the hydrogen atom in the 5'-hydroxy of the 5' terminal nucleotide of the sense strand or anti-sense strand, or a chemical moiety formed by removing the hydrogen atom in the 3'-hydroxy of the 3' terminal nucleotide of the sense strand or anti-sense strand.

On the basis of a total amount of siRNAs and siRNA groups in the first RNAi agent and the second RNAi agent respectively, a weight ratio of the first RNAi agent to the second RNAi agent in the pharmaceutical composition is 100: 1 to 1: 100, such as 100: 1, 50: 1, 25: 1, 15: 1, 10: 1, 5: 1, 4: 1, 3: 1, 2: 1, 1: 1, 1: 100, 1: 50, 1: 25, 1: 15, 1: 10, 1: 5, 1: 3, 1: 4 or 1: 2. In some embodiments, preferably, the weight ratio of the first RNAi agent to the second RNAi agent in the pharmaceutical composition is 10: 1 to 1: 10, 5: 1 to 1: 5 or 2: 1 to 1: 5. In some embodiments, in order to obtain a better treating/preventing effect, a weight of the second RNAi agent is not less than that of the first RNAi agent, and the weight ratio of the first RNAi agent to the second RNAi agent in the pharmaceutical composition is 1: 1 to 1: 10, 1: 1 to 1: 5 or 1: 1 to 1: 3.

In some embodiments, on the basis of the total amount of siRNAs and siRNA groups in the first RNAi agent and the second RNAi agent respectively, the weight of the first RNAi agent in the pharmaceutical composition is 1 mg-1000 mg, such as 1 mg-800 mg, 1 mg-600 mg, 1 mg-500 mg, 1 mg-300 mg, 1 mg-250 mg and 1 mg-100 mg. In some embodiments, the weight of the first RNAi agent is 5 mg-500 mg.

In some embodiments, the weight of the second RNAi agent is 1 mg-1000 mg, such as 1 mg-800 mg, 1 mg-600 mg, 1 mg-500 mg, 1 mg-300 mg, 1 mg-250 mg and 1 mg-100 mg. In some embodiments, the weight of the first RNAi agent in the pharmaceutical composition is 5 mg-500 mg, and in some embodiments, the weight of the second RNAi agent is 5 mg-800 mg.

In some embodiments, the pharmaceutical composition further comprises a third RNAi agent, the third RNAi agent comprises one or more third siRNAs and/or one or more third siRNA conjugates, each third siRNA is independently a siRNA capable of inhibiting *ANGPTL3* mRNA, and each third siRNA conjugate comprises a third siRNA group formed by one third siRNA and a conjugating group conjugated with the siRNA. In some embodiments, the third RNAi agent and the first RNAi agent and/or the second RNAi agent can further enhance the treating and/or preventing effects of the pharmaceutical composition of the present disclosure on the disease and/or symptom related to hyperlipidemia.

In some embodiments, weights of the third RNAi agent and the second RNAi agent are calculated on the basis of a total amount of siRNAs and siRNA groups in the third RNAi agent and the second RNAi agent respectively, a weight ratio of the third RNAi agent to the second RNAi agent in the pharmaceutical composition is 100: 1 to 1: 100, such as 100: 1, 50: 1, 25: 1, 15: 1, 10: 1, 5: 1, 4: 1, 3: 1, 2: 1, 1: 1, 1: 100, 1: 50, 1: 25, 1: 15, 1: 10, 1: 5, 1: 3, 1: 4 or 1: 2. In some embodiments, preferably, the weight ratio of the third RNAi agent to the second RNAi agent in the pharmaceutical composition is 10: 1 to 1: 10, 5: 1 to 1: 5 or 2: 1 to 1: 5.

In some embodiments, the weight of the third RNAi agent is 1 mg-1000 mg, such as 1 mg-800 mg, 1 mg-600 mg, 1 mg-500 mg, 1 mg-300 mg, 1 mg-250 mg or 1 mg-100 mg. In some embodiments, the weight of the third RNAi agent in the pharmaceutical composition is 5 mg-500 mg, and in some embodiments, the weight of the third RNAi agent is 5 mg-800 mg.

### First RNAi agent

As previously mentioned, the pharmaceutical composition of the present disclosure comprises the first siRNA agent. The first RNAi agent comprises one or more first siRNAs and/or one or more first siRNA conjugates, each first siRNA is independently a siRNA capable of inhibiting *APOC3* mRNA, and each first siRNA conjugate comprises a siRNA group formed by one first siRNA and a conjugating group conjugated with the siRNA.

The siRNA capable of inhibiting the *APOC3* mRNA may be any siRNA capable of inhibiting the *APOC3* mRNA. In some embodiments, each first siRNA comprises a sense strand and an anti-sense strand, and each nucleotide in the sense strand and the anti-sense strand is independently a modified or unmodified nucleotide; and a length of the sense strand is 18-23 nucleotides, a length of the anti-sense strand is 18-26 nucleotides, the sense strand and the anti-sense strand are basically or completely complementary in reverse, the first siRNA comprises at least one siRNA, and the sense strand of the first siRNA has at least 15 nucleotides continuously consistent with those in a nucleotide sequence shown in SEQ ID NO: 1, or the sense strand has at least 15 nucleotides continuously consistent with those in a nucleotide sequence shown in SEQ ID NO: 2:
5'-UGGCCUCCCAAUAAAGCUGGACAAGAAGCU-3' (SEQ ID NO: 1);
5'-UGCUUAAAAGGGACAGUAUUCUCAGUGCUC-3' (SEQ ID NO: 2).

In some embodiments, the sense strand has at least 15, 16, 17, 18 or 19 nucleotides continuously consistent with those in the nucleotide sequence shown in SEQ ID NO: 1 or SEQ ID NO: 2. In some embodiments, in a direction from 5' to 3', the sense strand comprises a nucleotide sequence in which nucleotides at positions 9-27 are continuously consistent with those in the nucleotide sequence shown in SEQ ID NO: 1, or comprises a nucleotide sequence in which nucleotides at positions 4-22 are continuously consistent with those in the nucleotide sequence shown in SEQ ID NO: 2.

The lengths of the sense strand and the anti-sense strand in the first siRNA may be the same or different. In some embodiments, a length ratio of the sense strand to the anti-sense strand of the first siRNA may be 19/19, 19/20, 19/21, 19/22, 19/23, 19/24, 19/25, 19/26, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25 or 23/26. For example, the length ratio of "19/19" means that the lengths of the sense strand and the anti-sense strand are both 19 nucleotides; the length ratio of "19/21" means that the length of the sense strand is 19 nucleotides, and the length of the anti-sense strand is 21 nucleotides; and so on. In some embodiments, the length ratio of the sense strand to the anti-sense strand of the first siRNA is 19/21, 21/23 or 23/25.

In some embodiments, in the first siRNA, some nucleotides in the sense strand and the anti-sense strand are modified nucleotides, and some nucleotides are unmodified nucleotides. In some embodiments, in the first siRNA, all nucleotides in the sense strand and the anti-sense strand are modified nucleotides; and according to a direction from a 5' terminal to a 3' terminal, 7^{th} to 9^{th} nucleotides of the sense strand are 2'-fluoro modified nucleotides, according to the direction from the 5' terminal to the 3' terminal, 2^{nd}, 6^{th}, 14^{th} and 16^{th} nucleotides of the anti-sense strand are 2'-fluoro modified nucleotides, and other nucleotides of the sense strand and the anti-sense strand are independently one of non-fluoro modified nucleotides.

In some embodiments, the first siRNA may be, for example, any siRNA listed in Table 1a and Table 1b, siRNA conjugates comprising these siRNAs or siRNA groups formed by these siRNAs show high *APOC3* mRNA inhibitory activity. In order to obtain a better inhibitory effect, in some embodiments, the first siRNA has a sequence shown as siAPOC3b1-M1SP1 and/or siAPOC3b3-M1S.

**Table 1a Sequence of first siRNA**

| siRNA No. | SEQ ID NO: | Sequence direction 5'- 3' |
|---|---|---|
| siAPOC3 b1 | 5 | UUAAAAGGGACAGUAUUCU |
| | 6 | AGAAUACUGUCCCUUUUAAGC |
| siAPOC3 b2 | 7 | GCUUAAAAGGGACAGUAUUCU |
| | 8 | AGAAUACUGUCCCUUUUAAGCAA |
| siAPOC3 b3 | 9 | UUAAAAGGGACAGUAUUCU |
| | 10 | AGAAUACUGUCCCUUUUAAUU |
| siAPOC3 b4 | 11 | GCUUAAAAGGGACAGUAUUCU |
| | 12 | AGAAUACUGUCCCUUUUAAGCUU |
| siAPOC3 b1-M1 | 13 | UmUmAmAmAmAmGfGfGfAmCmAmGmUmAmUmUmCmUm |
| | 14 | AmGfASAmUmAfCmUmGmUmCmCmCmUfUmUfUmAmAmGmCm |
| siAPOC3 b1-M2 | 15 | UmUmAmAmAmAmGfGfGfAmCmAmGmUmAmUmUmCmUm |
| | 16 | AmGfAmAmUSAfCmUmGmUmCmCmCmUfUmUfUmAmAmGmCm |
| siAPOC3 b2-M1 | 17 | GmCmUmUmAmAmAmAmGfGfGfAmCmAmGmUmAmUmUmCmUm |
| | 18 | AmGfASAmUmAfCmUmGmUmCmCmCmUfUmUfUmAmAmGmCmAm Am |
| siAPOC3 b2-M2 | 19 | GmCmUmUmAmAmAmAmGfGfGfAmCmAmGmUmAmUmUmCmUm |
| | 20 | AmGfAmAmUSAfCmUmGmUmCmCmCmUfUmUfUmAmAmGmCmAm Am |
| siAPOC3 b1-M1S | 21 | UmsUmsAmAmAmAmGfGfGfAmCmAmGmUmAmUmUmCmUm |
| | 22 | AmsGfsASAmUmAfCmUmGmUmCmCmCmUfUmUfUmAmAmsGmsCm |
| siAPOC3 b1-M2S | 23 | UmsUmsAmAmAmAmGfGfGfAmCmAmGmUmAmUmUmCmUm |
| | 24 | AmsGfsAmAmUSAfCmUmGmUmCmCmCmUfUmUfUmAmAmsGmsCm |
| siAPOC3 b2-M1S | 25 | GmsCmsUmUmAmAmAmAmGfGfGfAmCmAmGmUmAmUmUmCmUm |
| | 26 | AmsGfsASAmUmAfCmUmGmUmCmCmCmUfUmUfUmAmAmGmCms AmsAm |
| siAPOC3 b2-M2S | 27 | GmsCmsUmUmAmAmAmAmGfGfGfAmCmAmGmUmAmUmUmCmUm |
| | 28 | AmsGfsAmAmUSAfCmUmGmUmCmCmCmUfUmUfUmAmAmGmCms AmsAm |
| siAPOC3 b1-M1P1 | 29 | UmUmAmAmAmAmGfGfGfAmCmAmGmUmAmUmUmCmUm |
| | 30 | P1AmGfASAmUmAfCmUmGmUmCmCmCmUfUmUfUmAmAmGmCm |
| siAPOC3 b1-M2P1 | 31 | UmUmAmAmAmAmGfGfGfAmCmAmGmUmAmUmUmCmUm |
| | 32 | P1AmGfAmAmUSAfCmUmGmUmCmCmCmUfUmUfUmAmAmGmCm |
| siAPOC3 b2-M1P1 | 33 | GmCmUmUmAmAmAmAmGfGfGfAmCmAmGmUmAmUmUmCmUm |
| | 34 | P1AmGfASAmUmAfCmUmGmUmCmCmCmUfUmUfUmAmAmGmCm AmAm |
| siAPOC3 b2-M2P1 | 35 | GmCmUmUmAmAmAmAmGfGfGfAmCmAmGmUmAmUmUmCmUm |
| | 36 | P1AmGfAmAmUSAfCmUmGmUmCmCmCmUfUmUfUmAmAmGmCm AmAm |
| siAPOC3 b1-M1SP 1 | 37 | UmsUmsAmAmAmAmGfGfGfAmCmAmGmUmAmUmUmCmUm |
| | 38 | P1AmsGfsASAmUmAfCmUmGmUmCmCmCmUfUmUfUmAmAmsGms Cm |
| siAPOC3 b1-M2SP 1 | 39 | UmsUmsAmAmAmAmGfGfGfAmCmAmGmUmAmUmUmCmUm |
| | 40 | P1AmsGfsAmAmUSAfCmUmGmUmCmCmCmUfUmUfUmAmAmsGms Cm |
| siAPOC3 b2-M1SP 1 | 41 | GmsCmsUmUmAmAmAmAmGfGfGfAmCmAmGmUmAmUmUmCmUm |
| | 42 | P1AmsGfsASAmUmAfCmUmGmUmCmCmCmUfUmUfUmAmAmGmC msAmsAm |
| siAPOC3 b2-M2SP | 43 | GmsCmsUmUmAmAmAmAmGfGfGfAmCmAmGmUmAmUmUmCmUm |
| | 44 | P1AmsGfsAmAmUSAfCmUmGmUmCmCmCmUfUmUfUmAmAmGmC |
| 1 | | msAmsAm |
| siAPOC3 b3-M1 | 45 | UmUmAmAmAmAmGfGfGfAmCmAmGmUmAmUmUmCmUm |
| | 46 | AmGfASAmUmMAfCmUmGmMUmCmCmCmUfUmUfUnmAmMAmMUmUm |
| siAPOC3 b3-M2 | 47 | UmUmAmAmAmAmGfGfGfAmCmAmGmUmAmUmUmCmUm |
| | 48 | AmGfAmAmUSAfCmUmGmUmCmCmCmUfUmUfUmAmAmUmUm |
| siAPOC3 b4-M1 | 49 | GmCmUmUmAmAmAmAmGfGfGfAmCmAmGmUmAmUmUmCmUm |
| | 50 | AmGfASAmUmAfCmUmGmUmCmCmCmUfUmUfUmAmAmGmCmUm Um |
| siAPOC3 b4-M2 | 51 | GmCmUmUmAmAmAmAmGfGfGfAmCmAmGmUmAmUmUmCmUm |
| | 52 | AmGfAmAmUSAfCmUmGmUmCmCmCmUfUmUfUmAmAmGmCmUm Um |
| siAPOC3 b3-M1S | 53 | UmsUmsAmAmAmAmGfGfGfAmCmAmGmUmAmUmUmCmUm |
| | 54 | AmsGfsASAmUmAfCmUmGmUmCmCmCmUfUmUfUmAmAmsUmsU m |
| siAPOC3 b3-M2S | 55 | UmsUmsAmAmAmAmGfGfGfAmCmAmGmUmAmUmUmCmUm |
| | 56 | AmsGfsAmAmUSAfCmUmGmUmCmCmCmUfUmUfUmAmAmsUmsU m |
| siAPOC3 b4-M1S | 57 | GmsCmsUmUmAmAmAmAmGfGfGfAmCmAmGmUmAmUmUmCmUm |
| | 58 | AmsGfsASAmUmAfCmUmGmUmCmCmCmUfUmUfUmAmAmGmCms UmsUm |
| siAPOC3 b4-M2S | 59 | GmsCmsUmUmAmAmAmAmGfGfGfAmCmAmGmUmAmUmUmCmUm |
| | 60 | AmsGfsAmAmUSAfCmUmGmUmCmCmCmUfUmUfUmAmAmGmCms UmsUm |
| siAPOC3 b3-M1P1 | 61 | UmUmAmAmAmAmGfGfGfAmCmAmGmUmAmUmUmCmUm |
| | 62 | PlAmGfASAmUmAfCmUmGmUmCmCmCmUfUmUfUmAmAmUmUm |
| siAPOC3 b3-M2P1 | 63 | UmUmAmAmAmAmGfGfGfAmCmAmGmUmAmUmUmCmUm |
| | 64 | P1AmGfAmAmUSAfCmUmGmUmCmCmCmUfUmUfUmAmAmUmUm |
| siAPOC3 b4-M1P1 | 65 | GmCmUmUmAmAmAmAmGfGfGfAmCmAmGmUmAmUmUmCmUm |
| | 66 | P1AmGfASAmUmAfCmUmGmUmCmCmCmUfUmUfUmAmAmGmCm UmUm |
| siAPOC3 b4-M2P1 | 67 | GmCmUmUmAmAmAmAmGfGfGfAmCmAmGmUmAmUmUmCmUm |
| | 68 | P1AmGfAmAmUSAfCmUmGmUmCmCmCmUfUmUfUmAmAmGmCm UmUm |
| siAPOC3 b3-M1SP 1 | 69 | UmsUmsAmAmAmAmGfGfGfAmCmAmGmUmAmUmUmCmUm |
| | 70 | P1AmsGfsASAmUmAfCmUmGmUmCmCmCmUfUmUfUmAmAmsUms Um |
| siAPOC3 b3-M2SP 1 | 71 | UmsUmsAmAmAmAmGfGfGfAmCmAmGmUmAmUmUmCmUm |
| | 72 | P1AmsGfsAmAmUSAfCmUmGmUmCmCmCmUfUmUfUmAmAmsUms Um |
| siAPOC3 b4-M1SP 1 | 73 | GmsCmsUmUmAmAmAmAmGfGfGfAmCmAmGmUmAmUmUmCmUm |
| | 74 | P1AmsGfsASAmUmAfCmUmGmUmCmCmCmUfUmUfUmAmAmGmC msUmsUm |
| siAPOC3 b4-M2SP 1 | 75 | GmsCmsUmUmAmAmAmAmGfGfGfAmCmAmGmUmAmUmUmCmUm |
| | 76 | P1AmsGfsAmAmUSAfCmUmGmUmCmCmCmUfUmUfUmAmAmGmC msUmsUm |

**Table 1b Sequence of first siRNA**

| | | |
|---|---|---|
| siAP1 | 77 | CAAUAAAGCUGGACAAGAA |
| | 78 | UUCUUGUCCAGCUUUAUUGGG |
| siAP2 | 79 | CCCAAUAAAGCUGGACAAGAA |
| | 80 | UUCUUGUCCAGCUUUAUUGGGAG |
| siAP1-M1 | 81 | CmAmAmUmAfAmAfGfCfUmGmGmAmCmAmAmGmAmAm |
| | 82 | UmUfCmUmUmGfUmCfCfAmGmCmUmUfUmAfUmUmGmGmGm |
| siAP2-M1 | 83 | CmCmCmAmAmUmAfAmAfGfCfUmGmGmAmCmAmAmGmAmAm |
| | 84 | UmUfCmUmUmGfUmCfCfAmGmCmUmUfUmAfUmUmGmGmGmAm Gm |
| siAP1-M2 | 85 | CmAmAmUmAmAmAfGfCfUmGmGmAmCmAmAmGmAmAm |
| | 86 | UmUfCmUmUmGfUmCmCmAmGmCmUmUfUmAfUmUmGmGmGm |
| siAP2-M2 | 87 | CmCmCmAmAmUmAmAmAfGfCfUmGmGmAmCmAmAmGmAmAm |
| | 88 | UmUfCmUmUmGfUmCmCmAmGmCmUmUfUmAfUmUmGmGmGmA mGm |
| siAP1-M1 S | 89 | CmsAmsAmUmAfAmAfGfCfUmGmGmAmCmAmAmGmAmAm |
| | 90 | UmsUfsCmUmUmGfUmCfCfAmGmCmUmUfUmAfUmUmGmsGmsGm |
| siAP2-M1 S | 91 | CmsCmsCmAmAmUmAfAmAfGfCfUmGmGmAmCmAmAmGmAmAm |
| | 92 | UmsUfsCmUmUmGfUmCfCfAmGmCmUmUfUmAfUmUmGmGmGmsA msGm |
| siAP1-M2 S | 93 | CmsAmsAmUmAmAmAfGfCfUmGmGmAmCmAmAmGmAmAm |
| | 94 | UmsUfsCmUmUmGfUmCmCmAmGmCmUmUfUmAfUmUmGmsGmsG m |
| siAP2-M2 S | 95 | CmsCmsCmAmAmUmAmAmAfGfCfUmGmGmAmCmAmAmGmAmAm |
| | 96 | UmsUfsCmUmUmGfUmCmCmAmGmCmUmUfUmAfUmUmGmGmGms AmsGm |
| siAP1-M1 P1 | 97 | CmAmAmUmAfAmAfGfCfUmGmGmAmCmAmAmGmAmAm |
| | 98 | P1-UmUfCmUmUmGfUmCfCfAmGmCmUmUfUmAfUmUmGmGmGm |
| siAP2-M1 P1 | 99 | CmCmCmAmAmUmAfAmAfGfCfUmGmGmAmCmAmAmGmAmAm |
| | 100 | P1-UmUfCmUmUmGfUmCfCfAmGmCmUmUfUmAfUmUmGmGmGm AmGm |
| siAP1-M2 P1 | 101 | CmAmAmUmAmAmAfGfCfUmGmGmAmCmAmAmGmAmAm |
| | 102 | P1-UmUfCmUmUmGfUmCmCmAmGmCmUmUfUmAfUmUmGmGmG m |
| siAP2-M2 P1 | 103 | CmCmCmAmAmUmAmAmAfGfCfUmGmGmAmCmAmAmGmAmAm |
| | 104 | P1-UmUfCmUmUmGfUmCmCmAmGmCmUmUfUmAfUmUmGmGmG mAmGm |
| siAP1-M1 SP1 | 105 | CmsAmsAmUmAfAmAfGfCfUmGmGmAmCmAmAmGmAmAm |
| | 106 | P1-UmsUfsCmUmUmGfUmCfCfAmGmCmUmUfUmAfUmUmGmsGms Gm |

Wherein, capital letters C, G, U, and A indicate the base composition of the nucleotides; the lowercase m indicates that the nucleotide adjacent to the left side of the letter m is a methoxy modified nucleotide; the lowercase f indicates that the nucleotide adjacent to the left side of the letter f is a fluoro modified nucleotide; the underlined capital letter S indicates that the nucleotide adjacent to the left side of the letter S is a stably modified nucleotide; the lowercase s indicates that the two nucleotides adjacent to the left and right of the letter s are linked by phosphorothioate; and P1 represents that the nucleotide adjacent to the right side of P1 is a 5'-phosphate nucleotide or a 5'-phosphate analogue modified nucleotide. In some embodiments, S is a specific stably modified nucleotide such as moe, and the underlined letter combination moe indicates that the nucleotide adjacent to the left of the letter combination moe is a 2'-O-methoxyethyl modified nucleotide. In some embodiments, P1 is specifically modified VP, Ps or P, wherein the letter combination VP represents that the nucleotide adjacent to the right side of the letter combination VP is a 5'-(E)-vinylphosphonate (E-VP) modified nucleotide, the letter combination Ps represents that the nucleotide adjacent to the right side of the letter combination Ps is a phosphorothioate modified nucleotide, and the capital letter P represents that the nucleotide adjacent to the right side of the letter P is a 5'-phosphate nucleotide. In addition, each U in sequences listed in Tables 1a-1b above can be replaced by T, which will not significantly affect the activity or off-target effect of the siRNA.

In some embodiments, in each first siRNA conjugate, the conjugating group comprises a linker and a pharmaceutically acceptable targeting group and/or a delivery assisting group, and the first siRNA group, the linker, and the targeting group or the delivery assisting group are covalently or non-covalently linked in sequence, each targeting group is selected from ligands capable of binding with a cell surface receptor, and each delivery assisting group is selected from groups capable of enhancing the biocompatibility of the first siRNA conjugate in a delivery target organ or tissue.

In general, the conjugating group comprises at least one pharmaceutically acceptable targeting group and an optional linker. Moreover, the siRNA, the linker and the targeting group are linked in sequence. In some embodiments, there are 1-6 targeting groups. In some embodiments, there are 2-4 targeting groups. The siRNA group may be non-covalently or covalently conjugated to the conjugating group, for example, the siRNA group may be covalently conjugated to the conjugating group. The conjugating site between the siRNA group and the conjugating group may be at 3'-terminal or 5'-terminal of the sense strand of the siRNA group, or at 5'-terminal of the anti-sense strand, or within the internal sequence of the siRNA group. In some embodiments, the conjugating site between the siRNA group and the conjugating group is at 3' terminal of the sense strand.

In some embodiments, the conjugating group may be linked to the phosphate group, the 2'-hydroxy or the base in the siRNA group. In some embodiments, the conjugating group may be linked to the 3'-hydroxy when the nucleotides are linked via a 2'-5'-phosphodiester bond. When the conjugating group is linked to a terminal of the siRNA group, the conjugating group is typically linked to a phosphate group of a nucleotide; and when the conjugating group is linked to an internal sequence of the siRNA group, the conjugating group is typically linked to a ribose ring or a base. For specific linking modes, reference may be made to: Muthiah Manoharan *et.al.* siRNA conjugates carrying sequentially assembled trivalent N-acetylgalactosamine linked through nucleosides elicit robust gene silencing in vivo in hepatocytes. ACS Chemical biology, 2015, 10 (5): 1181-7.

In some embodiments, the siRNA group and the conjugating group may be linked by an acid labile or reducible chemical bond, and these chemical bonds may be degraded under the acidic environment of cell endosomes, thereby rendering the siRNA to be in free state, for example, the siRNA group may form the siRNA molecule again. For non degradable conjugating modes, the conjugating group may be linked to the sense strand of the siRNA group, thereby minimizing the effect of conjugating on the activity of the siRNA group.

The targeting group may be linked to the siRNA group via an appropriate linker, and the appropriate linker may be selected by those skilled in the art according to the specific type of the targeting group. The types of these linkers and targeting groups and the linking modes with the siRNA group may be found in the disclosure of WO2015006740A2, which is incorporated herein by reference in its entirety.

In some embodiments, the targeting group may be a conventionally used ligand in the field of siRNA administration, for example, the various ligands as described in WO2009082607A2, which is incorporated herein by reference in its entirety.

In some embodiments, at least one or each targeting group is selected from ligands capable of binding to a cell surface receptor expressing the target gene.

In some embodiments, at least one or each targeting group is selected from ligands capable of binding to a mammalian hepatic parenchymal cell surface receptor. In some embodiments, each targeting group is independently a ligand that has affinity to the asialoglycoprotein receptor on the surface of mammalian hepatocytes. In some embodiments, each targeting group is independently asialoglycoprotein or saccharide. In some embodiments, each targeting group is independently selected from one of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-α-neuraminic acid, 5-thio-β-D-glucofuranose, methyl 2,3,4-tris-O-acetyl-1-thio-6-0-trityl-α-D-glucofuranose, 4-thio-β-D-galactopyranose, ethyl 3,4,6,7-tetra-O-acetyl-2-deoxy-1,5-dithio-a-D-glucoheptopyranoside, 2,5-anhydro-D-allononitrile, ribose, D-ribose, D-4-thioribose, L-ribose, or L-4-thioribose. In some embodiments, at least one or each targeting group is galactose or N-acetylgalactosamine.

In some embodiments, the linker in the siRNA conjugate has a structure as shown by Formula (301): 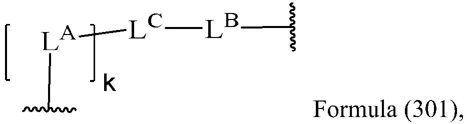
wherein, k is an integer of 1-3; and
L^{A} has an amide bond-comprising structure as shown by Formula (302), L^{B} has an N-acylpyrrolidine-comprising structure as shown by Formula (303), which comprises a carbonyl and an oxygen atom, and L^{C} is a linking group based on hydroxymethyl aminomethane, dihydroxymethyl aminomethane or trihydroxymethyl aminomethane: 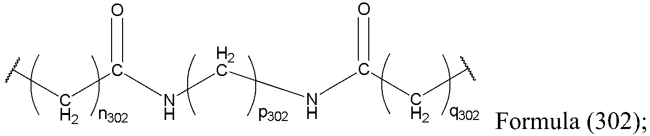 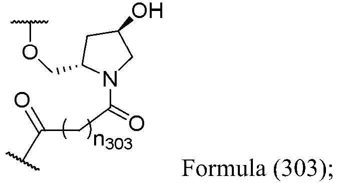
wherein, n₃₀₂, q₃₀₂ and p₃₀₂ are independently an integer of 2-6, and optionally, n₃₀₂, q₃₀₂ and p₃₀₂ are independently 2 or 3; n₃₀₃ is an integer of 4-16, and optionally, n₃₀₃ is an integer of 8-12; and 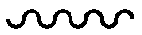 represents a site where the group is covalently linked.

In the linker, each L^{A} is respectively linked to one said targeting group through an ether bond, and linked through an ether bond formed by an oxygen atom of hydroxyl in the L^{C} moiety and the L^{C} moiety; and the L^{B} is linked through an amido bond formed by carbonyl in Formula (303) and a nitrogen atom of amino group in the L^{C} moiety, and linked through a phosphate bond or a thiophosphate bond formed by an oxygen atom in Formula (303) and an oxygen atom in the siRNA group.

In some embodiments, the siRNA conjugate has a structure as shown by Formula (305): 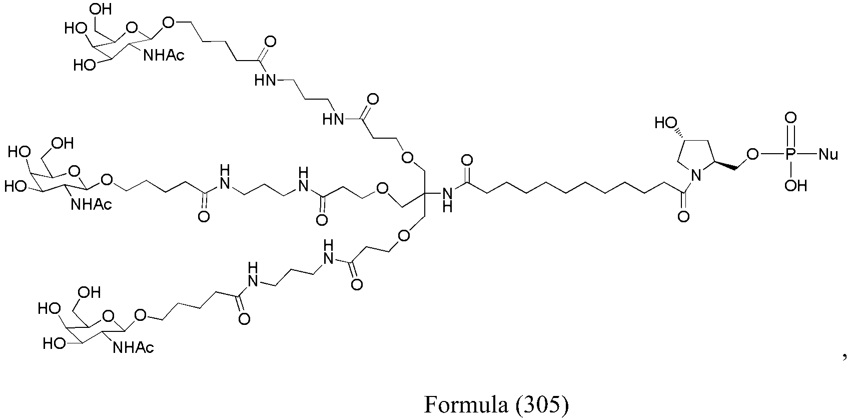 wherein, Nu represents the first siRNA group, the second siRNA group or the third siRNA group.

In some embodiments, the linker in the siRNA conjugate has a structure as shown by Formula (306): 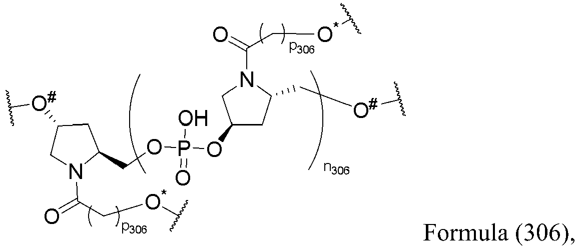 wherein, n₃₀₆ is an integer of 0-3, each p₃₀₆ is independently an integer of 1-6, and 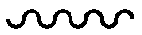 represents a site where the group is covalently linked; the linking group is linked through an ether bond formed by an oxygen atom marked by * and the targeting group; and the linking group is linked through a phosphate bond or a thiophosphate bond formed by at least one of oxygen atoms marked by # and the siRNA group, and the rest of the oxygen atoms marked by # are linked to hydrogen atoms to form hydroxyl groups, or linked to C₁-C₃ alkyl groups to form C₁-C₃ alkoxy groups.

In some embodiments, the siRNA conjugate has a structure as shown by Formula (307): 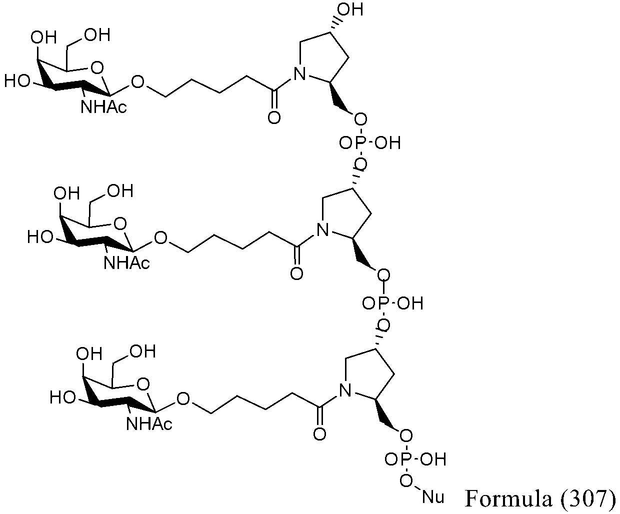 wherein, Nu represents the first siRNA group, the second siRNA group or the third siRNA group.

In some embodiments, the siRNA conjugate has a structure as shown by Formula (308): 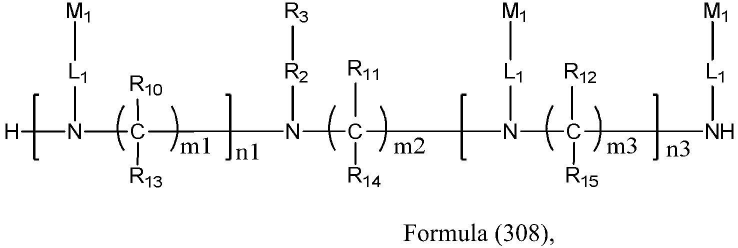 wherein,
n1 is an integer of 1-3, and n3 is an integer of 0-4;
each m1, m2 or m3 is independently an integer of 2-10;
R₁₀, R₁₁, R₁₂, R₁₃, R₁₄ or R₁₅ is independently H or selected from groups consisting of C₁-C₁₀ alkyl, C₁-C₁₀ haloalkyl and C₁-C₁₀ alkoxy; and
R₃ has a structure as shown by Formula A59: 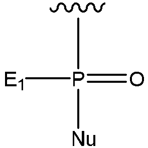 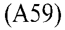
   wherein, L₁ is OH, SH or BH₂, and Nu indicates the first siRNA group, the second siRNA group or the third siRNA group;
   R₂ is a linear alkylene of 1-20 carbon atoms in length, wherein one or more carbon atoms are optionally replaced with any one or more of the group consisting of: C(O), NH, O, S, CH=N, S(O)₂, C₂-C₁₀ alkenylene, C₂-C₁₀ alkynylene, C₆-C₁₀ arylene, C₃-C₁₈ heterocyclylene, and C₅-C₁₀ heteroarylene; and wherein R₂ is optionally substituted by any one or more of the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halo substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl)(C₁-C₁₀ alkylphenyl), -NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), -CONH(C₁-C₁₀ alkyl), -CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, -SO₂NH(C₁-C₁₀ alkyl), -SO₂NH(phenyl), -NHSO₂(C₁-C₁₀ alkyl), -NHSO₂(phenyl), and-NHSO₂(C₁-C₁₀ haloalkyl);
   each L₁ is independently a linear alkylene of 1-70 carbon atoms in length, wherein one or more carbon atoms are optionally replaced with any one or more of the group consisting of: C(O), NH, O, S, CH=N, S(O)₂, C₂-C₁₀ alkenylene, C₂-C₁₀ alkynylene, C₆-C₁₀ arylene, C₃-C₁₈ heterocyclylene, and C₅-C₁₀ heteroarylene; and wherein L₁ is optionally substituted by any one or more of the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halo substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl)(C₁-C₁₀ alkylphenyl), -NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), -CONH(C₁-C₁₀ alkyl), -CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, -SO₂NH(C₁-C₁₀ alkyl), -SO₂NH(phenyl), -NHSO₂(C₁-C₁₀ alkyl), -NHSO₂(phenyl), and-NHSO₂(C₁-C₁₀ haloalkyl);
   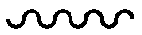 represents a site where the group is covalently linked; and
   M₁ represents a targeting group, of which the definitions and options are the same as those described above. In some embodiments, each M₁ is independently selected from one of the ligands that have affinity to the asialoglycoprotein receptor on the surface of mammalian hepatocytes.

Those skilled in the art would understand that, though L₁ is defined as linear alkylene for convenience, but it may not be a linear group or be named differently, such as amine or alkenyl produced by the above replacement and/or substitution. For the purpose of the present disclosure, the length of L₁ is the number of the atoms in the chain connecting the two attaching points. For this purpose, a ring obtained by replacement of a carbon atom of the linear alkylene (such as heterocyclylene or heteroarylene) is counted as one atom.

When M₁ is a ligand that has affinity to the asialoglycoprotein receptor on the surface of mammalian hepatocytes, in some embodiments, n1 may be an integer of 1-3, and n3 may be an integer of 0-4 to ensure that the number of the M₁ ligands in the conjugate may be at least 2. In some embodiments, n1+n3≥2, such that the number of the M₁ ligands may be at least 3, thereby allowing the M₁ ligand to more conveniently bind to the asialoglycoprotein receptor on the surface of hepatocytes, which may facilitate the endocytosis of the conjugate into cells. Experiments have shown that when the number of the M₁ ligands is greater than 3, the ease of binding the M₁ ligand to the asialoglycoprotein receptor on the surface of hepatocytes is not significantly increased. Therefore, in view of various aspects such as synthesis convenience, structure/process costs and delivery efficiency, in some embodiments, n1 is an integer of 1-2, n3 is an integer of 0-1, and n1+n3=2-3.

In some embodiments, when m1, m2 or m3 is independently selected from an integer of 2-10, the steric mutual positions among a plurality of M₁ ligands may be fit for binding the M₁ ligands to the asialoglycoprotein receptor on the surface of hepatocytes. In order to make the conjugate of the present disclosure have simpler structure, easier synthesis and/or reduced cost, in some embodiments, m1, m2 or m3 is independently an integer of 2-5, and in some embodiments, m1=m2=m3.

Those skilled in the art would understand that when R₁₀, R₁₁, R₁₂, R₁₃, R₁₄ or R₁₅ is independently selected from one of H, C₁-C₁₀ alkyl, C₁-C₁₀ haloalkyl, and C₁-C₁₀ alkoxy, they would not change the properties of the conjugate of the present disclosure and could all achieve the purpose of the present disclosure. In some embodiments, R₁₀, R₁₁, R₁₂, R₁₃, R₁₄ or R₁₅ is independently selected from selected from H, methyl or ethyl. In some embodiments, R₁₀, R₁₁, R₁₂, R₁₃, R₁₄ and R₁₅ are all H.

In some embodiments, R₃ is a group having the structure as shown by Formula A59, wherein E₁ is OH, SH or BH₂, and considering the availability of starting materials, in some embodiments, L₁ is OH or SH.

In some embodiments, R₂ is selected to achieve the linkage between the N atom on a nitrogenous backbone and the group as shown by Formula A59. In the context of the present disclosure, the "nitrogenous backbone" refers to a chain structure in which the carbon atoms attached to R₁₀, R₁₁, R₁₂, R₁₃, R₁₄, and R₁₅ and the N atoms are linked to each other. Therefore, R₂ may be any linking group capable of attaching the group as shown by Formula A59 to the N atom on a nitrogenous backbone by suitable means. In some embodiments, in the case where the siRNA conjugate is prepared by a solid phase synthesis process, the R₂ group needs to have both a site linking to the N atom on the nitrogenous backbone and a site linking to the P atom in R₃. In some embodiments, in R₂, the site linking to the N atom on the nitrogenous backbone forms an amide bond with the N atom, and the site linking to the P atom in R₃ forms a phosphoester bond with the P atom. In some embodiments, the length of R₂ is 4-15 atoms or 6-10 atoms. In some embodiments, R₂ may be B5, B6, B5' or B6': 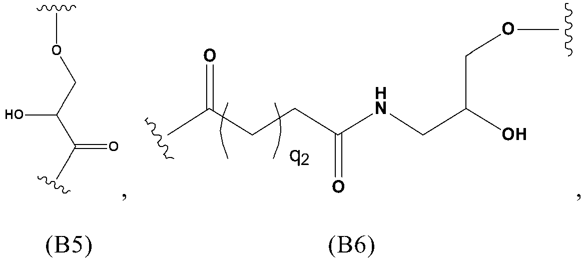 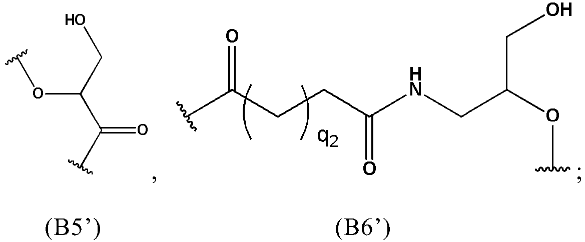 wherein, 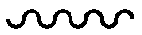 represents a site where a group is linked through a covalent bond.

A value range of q₂ may be an integer of 1-10; and in some embodiments, q₂ is an integer of *1-5.*

L₁ is used to link the M₁ ligand to the N atom on the nitrogenous backbone, thereby providing a targeting function for the siRNA conjugate. In some embodiments, L₁ is selected from the linking combinations of one or more of groups as shown by Formulae Al-A26. In some embodiments, L₁ is selected from the linking combinations of one or more of A1, A4, A5, A6, A8, A10, A11, and A13. In some embodiments, L₁ is selected from the linking combinations of at least two of A1, A4, A8, A10, and A11. In some embodiments, L₁ is selected from the linking combinations of at least two of A1, A8, and A10.

In some embodiments, the length of L₁ may be 3-25 atoms, 3-20 atoms, 4-15 atoms or 5-12 atoms. In some embodiments, the length of L₁ is 3, 4, 5, 6, 7, 8, 9, 10, 11, 12, 13, 14, 15, 16, 17, 18, 19, 20, 21, 22, 23, 24, 25, 30, 35, 40, 45, 50, 55, or 60 atoms.

In some embodiments, j1 is an integer of 2-10, and in some embodiments, j1 is an integer of 3-5. In some embodiments, j2 is an integer of 2-10, and in some embodiments, j2 is an integer of 3-5. R' is C₁-C₄ alkyl, and in some embodiments, R' is one of methyl, ethyl, and isopropyl. Ra is one of A27, A28, A29, A30, and A31, and in some embodiments, Ra is A27 or A28. Rb is C₁-C₅ alkyl, and in some embodiments, Rb is one of methyl, ethyl, isopropyl, and butyl. In some embodiments, j1, j2, R', Ra, and Rb of Formulae A1-A26 are respectively selected to achieve the linkage between the M₁ ligand and the N atom on the nitrogenous backbone, and to make the steric mutual positions among the M₁ ligands more suitable for binding the M₁ ligands to the asialoglycoprotein receptor on the surface of hepatocytes.

In some embodiments, the siRNA conjugate has a structure as shown by Formula (403), (404), (405), (406), (407), (408), (409), (410), (411), (412), (413), (414), (415), (416), (417), (418), (419), (420), (421) or (422):

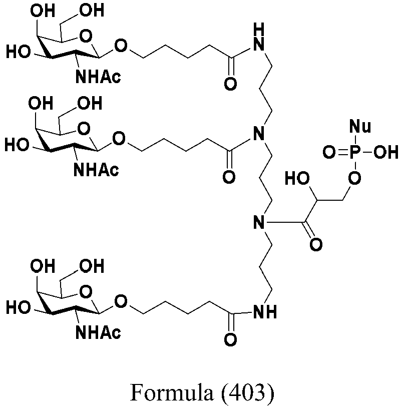

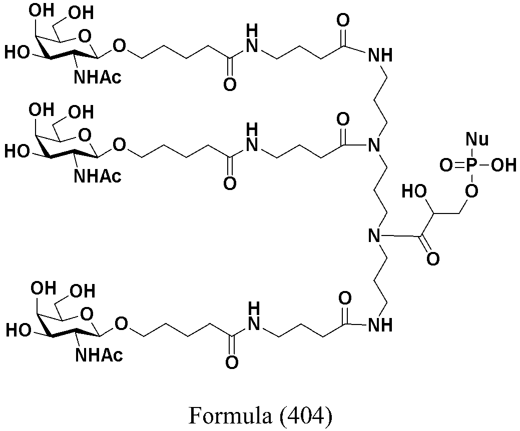

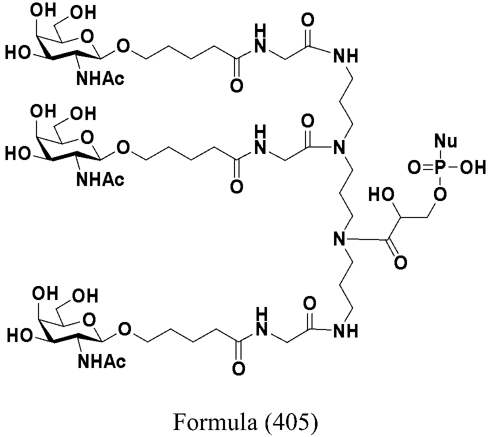

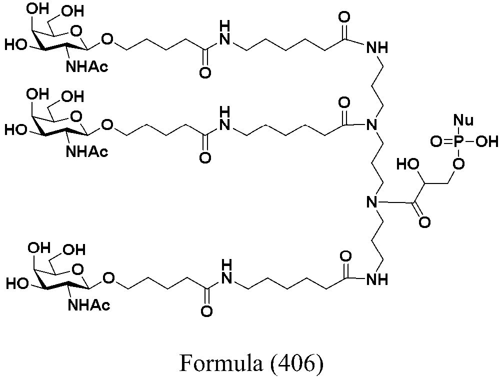

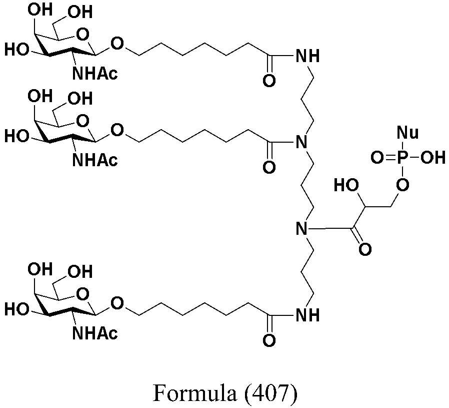

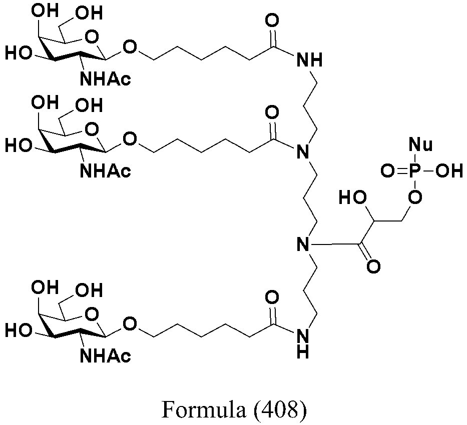

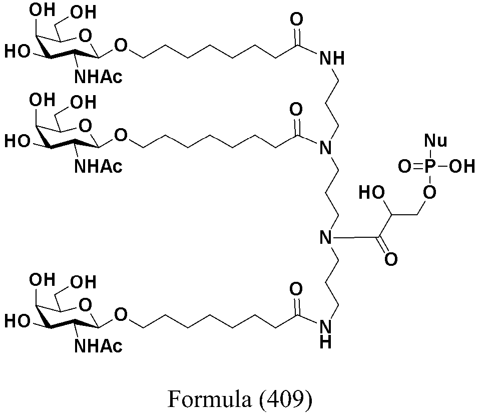

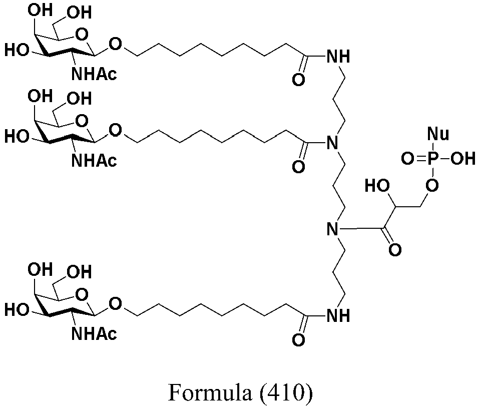

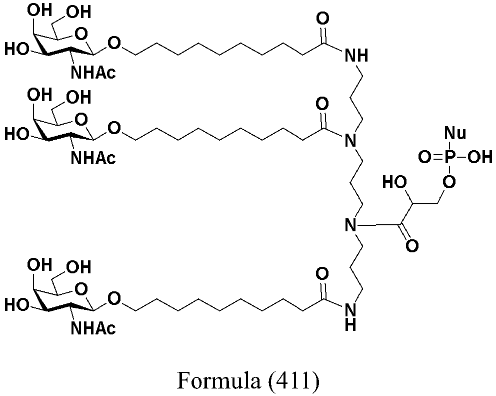

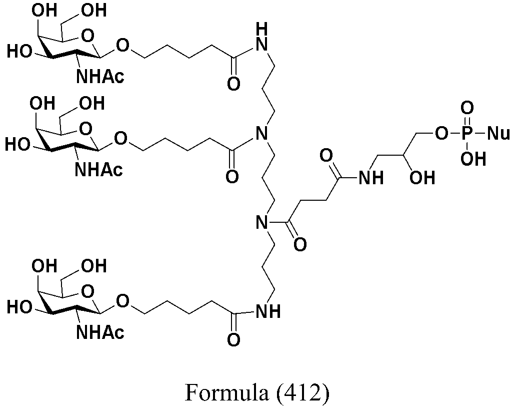

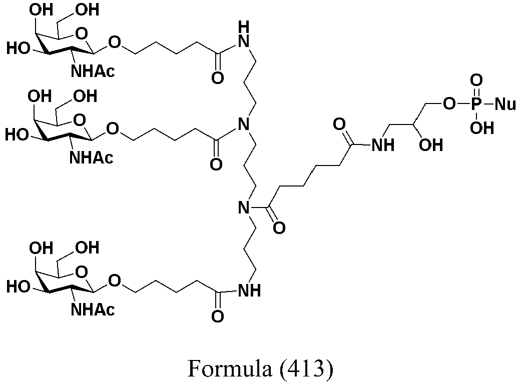

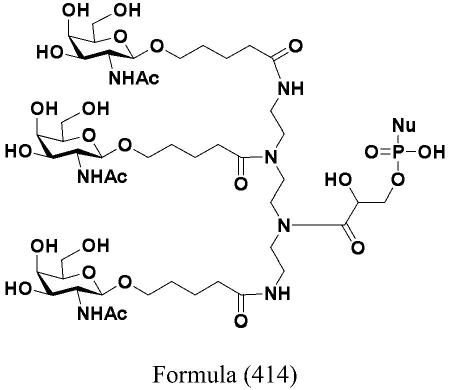

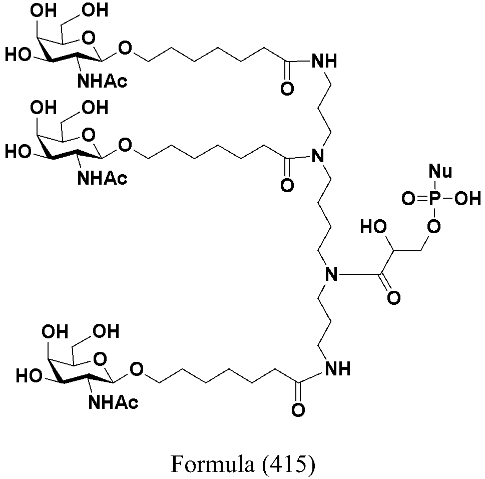

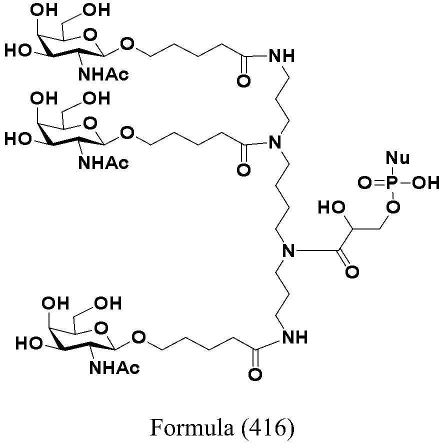

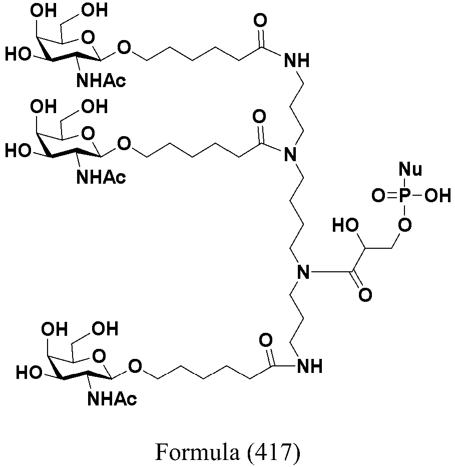

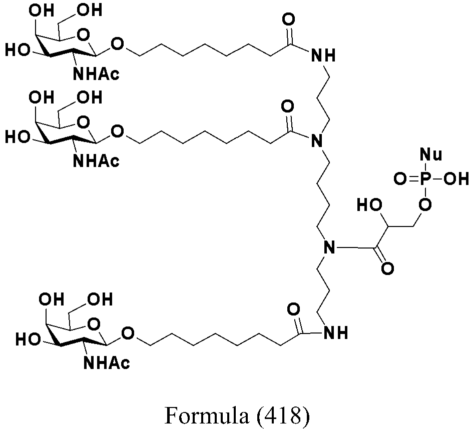

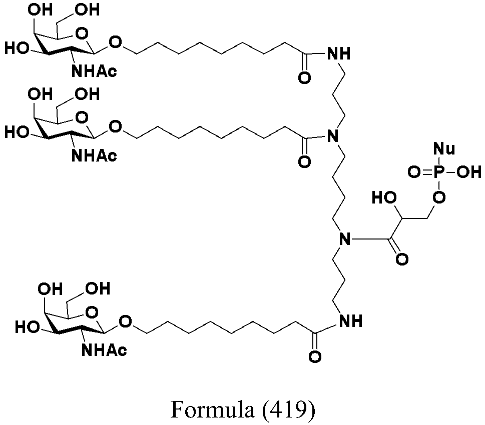

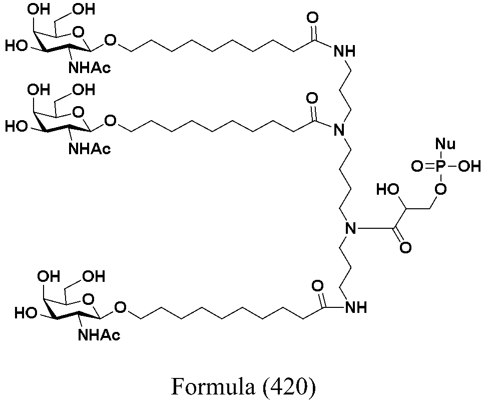

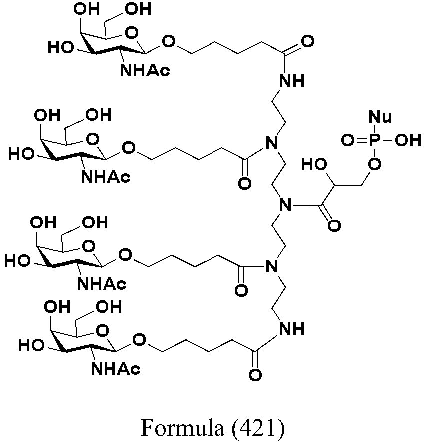

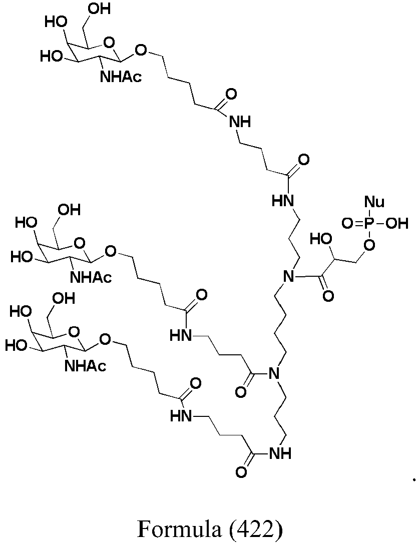

In some embodiments, the P atom in Formula A59 may be linked to any possible position in the siRNA group, for example, the P atom in Formula A59 may be linked to any nucleotide in the sense strand or the anti-sense strand of the siRNA group. In some embodiments, the P atom in Formula A59 is linked to any nucleotide in the sense strand. In some embodiments, the P atom in Formula A59 is linked to a terminal of the sense strand or the anti-sense strand. In some embodiments, the P atom in Formula A59 is linked to a terminal of the sense strand. The terminal refers to the first 4 nucleotides counted from one terminal of the sense strand or anti-sense strand. In some embodiments, the P atom in Formula A59 is linked to the terminal of the sense strand or the anti-sense strand of the siRNA group. In some embodiments, the P atom in Formula A59 is linked to 3' terminal of the sense strand. In the case of linkage to the above position in the sense strand, after entering into cells, the siRNA conjugate can release a separate anti-sense strand of the siRNA during unwinding, thereby adjusting targeting gene expression.

The P atom in Formula A59 may be linked to any possible position of a nucleotide in the siRNA group, for example, to position 5', 2' or 3' of the nucleotide, or to the base of the nucleotide. In some embodiments, the P atom in Formula A59 may be linked to position 2', 3', or 5' of the nucleotide in the siRNA group by forming a phosphodiester bond. In some embodiments, the P atom in Formula A59 is linked to an oxygen atom formed by dehydrogenation of the 3'-hydroxy of the nucleotide at 3' terminal of the sense strand, or the P atom in Formula A59 is linked to a nucleotide by substituting hydrogen in the 2'-hydroxy of the nucleotide of the sense strand, or the P atom in Formula A59 is linked to a nucleotide by substituting hydrogen in the 5'-hydroxy of the nucleotide at 5' terminal of the sense strand.

Those skilled in the art clearly know that a modified nucleotide group may be introduced into the siRNA of the present disclosure by a nucleoside monomer having a corresponding modification. The methods for preparing the nucleoside monomer having the corresponding modification and the methods for introducing the modified nucleotide group into the siRNA are also well-known to those skilled in the art. All the modified nucleoside monomers may be either commercially available or prepared by known methods.

The siRNA conjugate of the present disclosure may be prepared by any appropriate synthetic routes. For example, for a conjugating molecule comprising a targeting group and an active reaction group that can react with phosphoramidite to form a covalent linkage, the active group in the conjugating molecule may be protected by a protective agent first and then linked to a solid-phase carrier, and then the nucleoside monomers are linked one by one in the direction of 3' to 5' according to nucleotide types and sequences of the sense strand and the anti-sense strand of the siRNA by a phosphoramidite solid-phase synthesis method, and the linkage of each nucleoside monomer comprises four-step reaction of deprotection, coupling, capping, and oxidation or sulfurization. The sense strand and the anti-sense strand of the siRNA are separated and annealed to obtain the siRNA conjugate of the present disclosure.

Further, the preparation of the siRNA conjugate may also refer to the disclosures of existing documents. For example, a method for linking a linking group with a specific structure and a targeting ligand to a siRNA in sequence through reaction is described in Example 1 of WO2019010274A1, which is incorporated herein by reference in its entirety. For another example, a method for preparing various siRNA conjugates is described in detail in WO2015006740A2. For example, a method for preparing a structure as shown by Formula (305) is recorded in WO2014025805A1, and a method for preparing a structure as shown by Formula (307) is described Rajeev et al. in ChemBioChem 2015, 16, 903-908. The Chinese patent application CN110959011A also discloses a method for preparing a siRNA conjugate as shown by Formula (308) in detail. The contents of the above documents are incorporated herein by reference in its entirety.

The above description of the siRNA conjugate is also applicable to the second siRNA conjugate and the third siRNA conjugate below.

In the siRNA, the pharmaceutical composition or the siRNA conjugate of the present disclosure, the siRNA or the siRNA conjugate may exist in a salified form or an unsalified form. In the siRNA, the pharmaceutical composition or the siRNA conjugate of the present disclosure, the siRNA or the siRNA conjugate may be present in a form of partial or total water soluble salt, and the water soluble salt may be an amine salt or alkali metal salt thereof. In some embodiments, the alkali metal salt may be a potassium salt or a sodium salt. In some embodiments, the amine salt may be an ammonium salt, a methylamine salt or a triethylamine salt. In the first siRNA and/or the first siRNA conjugate, the second siRNA and/or the second siRNA conjugate, and the third siRNA and/or the third siRNA conjugate described in the context, each pair of adjacent nucleotides is linked via a phosphodiester bond or phosphorothioate diester bond. The non-bridging oxygen atom or sulfur atom in the phosphodiester bond or phosphorothioate diester bond is negatively charged, and may be present in the form of hydroxy or sulfhydryl. Moreover, the hydrogen ion in the hydroxy or sulfhydryl may be partially or completely substituted with a cation. The cation may be any cation, such as a metal cation, an ammonium ion NH₄⁺ or an organic ammonium cation. The organic ammonium cation may be a cation formed by methylamine, a cation formed by triethylamine or a quaternary ammonium cation. In order to increase solubility, in some embodiments, the cation is selected from one or more of an alkali metal ion, an ammonium cation formed by a tertiary amine and a quaternary ammonium cation. The alkali metal ion may be K⁺ and/or Na⁺, and the cation formed by the tertiaryamine may be an ammonium ion formed by triethylamine and/or an ammonium ion formed by N,N-diisopropylethylamine. Thus, the siRNA or the siRNA conjugate of the present disclosure may be at least partially present in the form of salt. In one embodiment, the non-bridging oxygen atom or sulfur atom in the phosphodiester bond or the phosphorothioate diester bond at least partially binds to a sodium ion, and thus the siRNA or the siRNA conjugate is present in a form of sodium salt or partial sodium salt.

In some embodiments, the first RNAi agent comprises 2-4 first siRNAs and/or first siRNA conjugates, wherein a content of each first siRNA and/or first siRNA conjugate is equal or unequal. In some embodiments, the first RNAi agent comprises 2 first siRNAs and/or first siRNA conjugates, a ratio between them may 0.1-10, such as 0.4-5.

In some embodiments, for simple preparation, the first RNAi agent only comprises one first siRNA or first siRNA conjugate. In some embodiments, in order to obtain better delivery effect, the first RNAi agent only comprises one first siRNA conjugate.

### Second RNAi agent in pharmaceutical composition of the present disclosure

In the context of the present disclosure, the second RNAi agent comprises one or more second siRNAs and/or one or more second siRNA conjugates, each second siRNA is independently a siRNA capable of inhibiting *PCSK9* mRNA, and each second siRNA conjugate comprises a siRNA group formed by one second siRNA and a conjugating group conjugated with the siRNA.

The siRNA capable of inhibiting the *PCSK9* mRNA may be any siRNA capable of inhibiting the *PCSK9* mRNA. In some embodiments, each second siRNA comprises a sense strand and an anti-sense strand, and each nucleotide in the sense strand and the anti-sense strand is independently a modified or unmodified nucleotide; and a length of the sense strand is 18-23 nucleotides, a length of the anti-sense strand is 18-26 nucleotides, the sense strand and the anti-sense strand are basically or completely complementary in reverse, the second siRNA comprises at least one siRNA, and the sense strand of the second siRNA has at least 15 nucleotides continuously consistent with those in a nucleotide sequence shown in SEQ ID NO: 3:
5'-UUCUAGACCUGUUUUGCUUUUGUAACUUGA-3' (SEQ ID NO: 3).

In some embodiments, the sense strand of the siRNA has at least 15, 16, 17, 18 or 19 nucleotides continuously consistent with those in the nucleotide sequence shown in SEQ ID NO: 3. In some embodiments, in a direction from 5' to 3', the sense strand comprises a nucleotide sequence in which nucleotides at positions 9-27 are continuously consistent with those in the nucleotide sequence shown in SEQ ID NO: 3.

The lengths of the sense strand and the anti-sense strand in the second siRNA may be the same or different. In some embodiments, a length ratio of the sense strand to the anti-sense strand of the second siRNA may be 19/19, 19/20, 19/21, 19/22, 19/23, 19/24, 19/25, 19/26, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25 or 23/26. In some embodiments, the length ratio of the sense strand to the anti-sense strand of the second siRNA is 19/21, 21/23 or 23/25.

In some embodiments, in the second siRNA, some nucleotides in the sense strand and the anti-sense strand are modified nucleotides, and some nucleotides are unmodified nucleotides. In some embodiments, in the second siRNA, all nucleotides in the sense strand and the anti-sense strand are modified nucleotides; and according to a direction from a 5' terminal to a 3' terminal, 7^{th} to 9^{th} nucleotides of the sense strand are 2'-fluoro modified nucleotides, according to the direction from the 5' terminal to the 3' terminal, 2^{nd} 6^{th}, 14^{th} and 16^{th} nucleotides of the anti-sense strand are 2'-fluoro modified nucleotides, and other nucleotides of the sense strand and the anti-sense strand are independently one of non-fluoro modified nucleotides.

In some embodiments, the second siRNA may be, for example, any siRNA listed in Table 2, siRNA conjugates comprising these siRNAs or siRNA groups formed by these siRNAs show high *PCSK9* mRNA inhibitory activity. In order to obtain a better inhibitory effect, in some embodiments, the second siRNA has a sequence shown as siPCSKd1-M1S.

**Table 2. Sequence of second siRNA**

| siRNA No. | SEQ ID NO: | Sequence direction 5'- 3' |
|---|---|---|
| siPCSKd1 | 107 | CUGUUUUGCUUUUGUAACU |
| | 108 | AGUUACAAAAGCAAAACAGGU |
| siPCSKd2 | 109 | ACCUGUUUUGCUUUUGUAACU |
| | 110 | AGUUACAAAAGCAAAACAGGUCU |
| siPCSKd1 -M1 | 111 | CmUmGmUmUmUmUfGfCfUmUmUmUmGmUmAmAmCmUm |
| | 112 | AmGfUmUmAmCfAmAmAmAmGmCmAmAfAmAfCmAmGmGmUm |
| siPCSKd1 -M2 | 113 | CmUmGmUmUfUmUfGfCfUmUmUmUmGmUmAmAmCmUm |
| | 114 | AmGfUmUmAmCfAmAfAfAmGmCmAmAfAmAfCmAmGmGmUm |
| siPCSKd1 -M3 | 115 | CmUmGmUmUfUmUfGfCfUmUmUmUmGmUmAmAmCmUm |
| | 116 | AmGfUmUmAmCfAmAmAmAmGmCmAmAfAmAfCmAmGmGmUm |
| siPCSKd2 -M1 | 117 | AmCmCmUmGmUmUmUmUfGfCfUmUmUmUmGmUmAmAmCmUm |
| | 118 | AmGfUmUmAmCfAmAmAmAmGmCmAmAfAmAfCmAmGmGmUmC mUm |
| siPCSKd2 -M2 | 119 | AmCmCmUmGmUmUfUmUfGfCfUmUmUmUmGmUmAmAmCmUm |
| | 120 | AmGfUmUmAmCfAmAfAfAmGmCmAmAfAmAfCmAmGmGmUmCm Um |
| siPCSKd2 -M3 | 121 | AmCmCmUmGmUmUfUmUfGfCfUmUmUmUmGmUmAmAmCmUm |
| | 122 | AmGfUmUmAmCfAmAmAmAmGmCmAmAfAmAfCmAmGmGmUmC mUm |
| siPCSKd1 -M1S | 123 | CmsUmsGmUmUmUmUfGfCfUmUmUmUmGmUmAmAmCmUm |
| | 124 | AmsGfsUmUmAmCfAmAmAmAmGmCmAmAfAmAfCmAmGmsGmsU m |
| siPCSKd1 -M2 S | 125 | CmsUmsGmUmUfUmUfGfCfUmUmUmUmGmUmAmAmCmUm |
| | 126 | AmsGfsUmUmAmCfAmAfAfAmGmCmAmAfAmAfCmAmGmsGmsUm |
| siPCSKd1 -M3S | 127 | CmsUmsGmUmUfUmUfGfCfUmUmUmUmGmUmAmAmCmUm |
| | 128 | AmsGfsUmUmAmCfAmAmAmAmGmCmAmAfAmAfCmAmGmsGmsU m |
| siPCSKd2 -M1S | 129 | AmsCmsCmUmGmUmUmUmUfGfCfUmUmUmUmGmUmAmAmCmUm |
| | 130 | AmsGfsUmUmAmCfAmAmAmAmGmCmAmAfAmAfCmAmGmGmUm sCmsUm |
| siPCSKd2 -M2S | 131 | AmsCmsCmUmGmUmUfUmUfGfCfUmUmUmUmGmUmAmAmCmUm |
| | 132 | AmsGfsUmUmAmCfAmAfAfAmGmCmAmAfAmAfCmAmGmGmUmsC msUm |
| siPCSKd2 -M3S | 133 | AmsCmsCmUmGmUmUfUmUfGfCfUmUmUmUmGmUmAmAmCmUm |
| | 134 | AmsGfsUmUmAmCfAmAmAmAmGmCmAmAfAmAfCmAmGmGmUm sCmsUm |
| siPCSKd1 -M1P1 | 135 135 | CmUmGmUmUmUmUfGfCfUmUmUmUmGmUmAmAmCmUm |
| | 136 | P1AmGfUmUmAmCfAmAmAmAmGmCmAmAfAmAfCmAmGmGmUm |
| siPCSKd1 -M2P1 | 137 | CmUmGmUmUfUmUfGfCfUmUmUmUmGmUmAmAmCmUm |
| | 138 | P1AmGfUmUmAmCfAmAfAfAmGmCmAmAfAmAfCmAmGmGmUm |
| siPCSKd1 -M3P1 | 139 | CmUmGmUmUfUmUfGfCfUmUmUmUmGmUmAmAmCmUm |
| | 140 | P1AmGfUmUmAmCfAmAmAmAmGmCmAmAfAmAfCmAmGmGmUm |
| siPCSKd2 -M1P1 | 141 | AmCmCmUmGmUmUmUmUfGfCfUmUmUmUmGmUmAmAmCmUm |
| | 142 | P1AmGfUmUmAmCfAmAmAmAmGmCmAmAfAmAfCmAmGmGmUm CmUm |
| siPCSKd2 -M2P1 | 143 | AmCmCmUmGmUmUfUmUfGfCfUmUmUmUmGmUmAmAmCmUm |
| | 144 | P1AmGfUmUmAmCfAmAfAfAmGmCmAmAfAmAfCmAmGmGmUmC |
| | | mUm |
| siPCSKd2 -M3P1 | 145 | AmCmCmUmGmUmUfUmUfGfCfUmUmUmUmGmUmAmAmCmUm |
| | 146 | P1AmGfUmUmAmCfAmAmAmAmGmCmAmAfAmAfCmAmGmGmUm CmUm |
| siPCSKd1 -M1SP1 | 147 | CmsUmsGmUmUmUmUfGfCfUmUmUmUmGmUmAmAmCmUm |
| | 148 | P1AmsGfsUmUmAmCfAmAmAmAmGmCmAmAfAmAfCmAmGmsGm sUm |
| siPCSKd1 -M2SP1 | 149 | CmsUmsGmUmUfUmUfGfCfUmUmUmUmGmUmAmAmCmUm |
| | 150 | P1AmsGfsUmUmAmCfAmAfAfAmGmCmAmAfAmAfCmAmGmsGmsU m |
| siPCSKd1 -M3SP1 | 151 | CmsUmsGmUmUfUmUfGfCfUmUmUmUmGmUmAmAmCmUm |
| | 152 | P1AmsGfsUmUmAmCfAmAmAmAmGmCmAmAfAmAfCmAmGmsGm sUm |
| siPCSKd2 -M1SP1 | 153 | AmsCmsCmUmGmUmUmUmUfGfCfUmUmUmUmGmUmAmAmCmUm |
| | 154 | P1AmsGfsUmUmAmCfAmAmAmAmGmCmAmAfAmAfCmAmGmGmU msCmsUm |
| siPCSKd2 -M2SP1 | 155 | AmsCmsCmUmGmUmUfUmUfGfCfUmUmUmUmGmUmAmAmCmUm |
| | 156 | P1AmsGfsUmUmAmCfAmAfAfAmGmCmAmAfAmAfCmAmGmGmUm sCmsUm |
| siPCSKd2 -M3SP1 | 157 | AmsCmsCmUmGmUmUfUmUfGfCfUmUmUmUmGmUmAmAmCmUm |
| | 158 | P1AmsGfsUmUmAmCfAmAmAmAmGmCmAmAfAmAfCmAmGmGmU msCmsUm |

Wherein, capital letters C, G, U, and A indicate the base composition of the nucleotides; the lowercase m indicates that the nucleotide adjacent to the left side of the letter m is a methoxy modified nucleotide; the lowercase f indicates that the nucleotide adjacent to the left side of the letter f is a fluoro modified nucleotide; the lowercase s indicates that the two nucleotides adjacent to the left and right of the letter s are linked by phosphorothioate; and P1 represents that the nucleotide adjacent to the right side of P1 is a 5'-phosphate nucleotide or a 5'-phosphate analogue modified nucleotide. In some embodiments, P1 is specifically modified VP, Ps or P, wherein the letter combination VP represents that the nucleotide adjacent to the right side of the letter combination VP is a 5'-(E)-vinylphosphonate (E-VP) modified nucleotide, the letter combination Ps represents that the nucleotide adjacent to the right side of the letter combination Ps is a phosphorothioate modified nucleotide, and the capital letter P represents that the nucleotide adjacent to the right side of the letter P is a 5'-phosphate nucleotide. In addition, each U in sequences listed in Table 2 above can be replaced by T, which will not significantly affect the activity or off-target effect of the siRNA.

In some embodiments, in each second siRNA conjugate, the conjugating group comprises a linker and a pharmaceutically acceptable targeting group and/or a delivery assisting group, and the second siRNA group, the linker, and the targeting group or the delivery assisting group are covalently or non-covalently linked in sequence. The definition and option of the targeting group are the same as those of the targeting group in the first siRNA conjugate described above. In some embodiments, the second siRNA conjugate has a structure as shown by Formula (403).

In some embodiments, the second RNAi agent comprises 2-4 second siRNAs and/or second siRNA conjugates, wherein a content of each second siRNA and/or second siRNA conjugate is equal or unequal. In some embodiments, the second RNAi agent comprises 2 second siRNAs and/or second siRNA conjugates, a ratio between them may 0.1-10, such as 0.4-5.

In some embodiments, for simple preparation, the second RNAi agent only comprises one second siRNA or second siRNA conjugate. In some embodiments, in order to obtain better delivery effect, the second RNAi agent only comprises one second siRNA conjugate.

In some embodiments, in the pharmaceutical composition provided by the present disclosure, the first RNAi agent only comprises one first siRNA conjugate and/or the second RNAi agent only comprises one second siRNA conjugate; the sense strand and the anti-sense strand of the first siRNA are basically or completely complementary in reverse, and the sense strand of the first siRNA has at least 15 nucleotides continuously consistent with those in the nucleotide sequence shown in SEQ ID NO: 1, or the sense strand of the second siRNA has at least 15 nucleotides continuously consistent with those in the nucleotide sequence shown in SEQ ID NO: 2; and the sense strand and the anti-sense strand of the second siRNA are basically or completely complementary in reverse, and the sense strand has at least 15 nucleotides continuously consistent with those in the nucleotide sequence shown in SEQ ID NO: 3;
5'-UGGCCUCCCAAUAAAGCUGGACAAGAAGCU-3' (SEQ ID NO: 1);
5'-UGCUUAAAAGGGACAGUAUUCUCAGUGCUC-3' (SEQ ID NO: 2);
5'-UUCUAGACCUGUUUUGCUUUUGUAACUUGA-3' (SEQ ID NO: 3).

### Third RNAi agent in pharmaceutical composition of the present disclosure

In some embodiments, the pharmaceutical composition of the present disclosure further comprises a third RNAi agent, wherein the third RNAi agent comprises one or more third siRNAs and/or one or more third siRNA conjugates, each third siRNA is independently a siRNA capable of inhibiting *ANGPTL3* mRNA, and each third siRNA conjugate comprises a third siRNA group formed by one third siRNA and a conjugating group conjugated with the siRNA. In some embodiments, the third RNAi agent and the first RNAi agent and/or the second RNAi agent have a synergistic effect, and can further enhance the treating and/or preventing effects of the pharmaceutical composition of the present disclosure on the disease and/or symptom related to hyperlipidemia.

The siRNA capable of inhibiting the *ANGPTL3* mRNA may be any siRNA capable of inhibiting the *ANGPTL3* mRNA. In some embodiments, each third siRNA comprises a sense strand and an anti-sense strand, and each nucleotide in the sense strand and the anti-sense strand is independently a modified or unmodified nucleotide; and a length of the sense strand is 18-23 nucleotides, a length of the anti-sense strand is 18-26 nucleotides, the sense strand and the anti-sense strand are basically or completely complementary in reverse, the third siRNA comprises at least one siRNA, and the sense strand of the third siRNA has at least 15 nucleotides continuously consistent with those in a nucleotide sequence shown in SEQ ID NO: 4:
5'-AGCCAAAAUCAAGAUUUGCUAUGUUAGACG-3' (SEQ ID NO: 4).

In some embodiments, the sense strand of the siRNA has at least 15, 16, 17, 18 or 19 nucleotides continuously consistent with those in the nucleotide sequence shown in SEQ ID NO: 4. In some embodiments, in a direction from 5' to 3', the sense strand comprises a nucleotide sequence in which nucleotides at positions 9-27 are continuously consistent with those in the nucleotide sequence shown in SEQ ID NO: 4, or comprises a nucleotide sequence in which no more than 1 nucleotide is different from nucleotides at positions 9-27 in the nucleotide sequence shown in SEQ ID NO: 4.

The lengths of the sense strand and the anti-sense strand in the third siRNA may be the same or different. In some embodiments, a length ratio of the sense strand to the anti-sense strand of the third siRNA may be 19/19, 19/20, 19/21, 19/22, 19/23, 19/24, 19/25, 19/26, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25 or 23/26. In some embodiments, the length ratio of the sense strand to the anti-sense strand of the second siRNA is 19/21, 21/23 or 23/25.

In some embodiments, in the third siRNA, some nucleotides in the sense strand and the anti-sense strand are modified nucleotides, and some nucleotides are unmodified nucleotides. In some embodiments, in the third siRNA, all nucleotides in the sense strand and the anti-sense strand are modified nucleotides; and according to a direction from a 5' terminal to a 3' terminal, 7^{th} to 9^{th} nucleotides of the sense strand are 2'-fluoro modified nucleotides, according to the direction from the 5' terminal to the 3' terminal, 2^{nd}, 6^{th}, 14^{th} and 16^{th} nucleotides of the anti-sense strand are 2'-fluoro modified nucleotides, and other nucleotides of the anti-sense strand are independently one of non-fluoro modified nucleotides.

In some embodiments, the third siRNA may be, for example, any siRNA listed in Table 3, siRNA conjugates comprising these siRNAs or siRNA groups formed by these siRNAs show high *ANGPTL3* mRNA inhibitory activity. In order to obtain a better inhibitory effect, in some embodiments, the third siRNA has a sequence shown as siANGa1-M1S or siANGa1-M2S.

**Table 3 Sequence of third siRNA**

| siRNA No. | SEQ ID NO: | Sequence direction 5'- 3' |
|---|---|---|
| siANGa1 | 159 | AAAUCAAGAUUUGCUAUGU |
| | 160 | ACAUAGCAAAUCUUGAUUUUG |
| siANGa2 | 161 | CAAAAUCAAGAUUUGCUAUGU |
| | 162 | ACAUAGCAAAUCUUGAUUUUGGC |
| siANGa1-M1 | 163 | AmAmAmUmCmAmAfGfAfUmUmUmGmCmUmAmUmGmUm |
| | 164 | AmCfASUmAmGfCmAmAmAmUmCmUmUfGmAfUmUmUmUmGm |
| siANGa1-M2 | 165 | AmAmAmUmCmAmAfGfAfUmUmUmGmCmUmAmUmGmUm |
| | 166 | AmCfAmUmASGfCmAmAmAmUmCmUmUfGmAfUmUmUmUmGm |
| siANGa2-M1 | 167 | AmAmAmUmCmAmAfGfAfUmUmUmGmCmUmAmUmCmUm |
| | 168 | AmCfASUmAmGfCmAmAmAmUmCmUmUfGmAfUmUmUmUmGm |
| siANGa2-M2 | 169 | AmAmAmUmCmAmAfGfAfUmUmUmGmCmUmAmUmCmUm |
| | 170 | AmCfAmUmASGfCmAmAmAmUmCmUmUfGmAfUmUmUmUmGm |
| siANGa3-M1 | 171 | AmAmAmUmCmAmAfGfAfUmUmUmGmCmUmAmUmAmUm |
| | 172 | AmCfASUmAmGfCmAmAmAmUmCmUmUfGmAfUmUmUmUmGm |
| siANGa3-M2 | 173 | AmAmAmUmCmAmAfGfAfUmUmUmGmCmUmAmUmAmUm |
| | 174 | AmCfAmUmASGfCmAmAmAmUmCmUmUfGmAfUmUmUmUmGm |
| siANGa4-M1 | 175 | AmAmAmUmCmAmAfGfAfUmUmUmGmCmUmAmUmUmUm |
| | 176 | AmCfASUmAmGfCmAmAmAmUmCmUmUfGmAfUmUmUmUmGm |
| siANGa4-M2 | 177 | AmAmAmUmCmAmAfGfAfUmUmUmGmCmUmAmUmUmUm |
| | 178 | AmCfAmUmASGfCmAmAmAmUmCmUmUfGmAfUmUmUmUmGm |
| siANGa5-M1 | 179 | CmAmAmAmAmUmCmAmAfGfAfUmUmUmGmCmUmAmUmGmUm |
| | 180 | AmCfASUmAmGfCmAmAmAmUmCmUmUfGmAfUmUmUmUmGmGmC m |
| siANGa5-M2 | 181 | CmAmAmAmAmUmCmAmAfGfAfUmUmUmGmCmUmAmUmGmUm |
| | 182 | AmCfAmUmASGfCmAmAmAmUmCmUmUfGmAfUmUmUmUmGmGmC m |
| siANGa6-M1 | 183 | CmAmAmAmAmUmCmAmAfGfAfUmUmUmGmCmUmAmUmCmUm |
| | 184 | AmCfAS̲UmAmGfCmAmAmAmUmCmUmUfGmAfUmUmUmUmGmGmC m |
| siANGa6-M2 | 185 | CmAmAmAmAmUmCmAmAfGfAfUmUmUmGmCmUmAmUmCmUm |
| | 186 | AmCfAmUmASGfCmAmAmAmUmCmUmUfGmAfUmUmUmUmGmGmC m |
| siANGa7-M1 | 187 | CmAmAmAmAmUmCmAmAfGfAfUmUmUmGmCmUmAmUmAmUm |
| | 188 | AmCfASUmAmGfCmAmAmAmUmCmUmUfGmAfUmUmUmUmGmGmC m |
| siANGa7-M2 | 189 | CmAmAmAmAmUmCmAmAfGfAfUmUmUmGmCmUmAmUmAmUm |
| | 190 | AmCfAmUmASGfCmAmAmAmUmCmUmUfGmAfUmUmUmUmGmGmC m |
| siANGa8-M1 | 191 | CmAmAmAmAmUmCmAmAfGfAfUmUmUmGmCmUmAmUmUmUm |
| | 192 | AmCfASUmAmGfCmAmAmAmUmCmUmUfGmAfUmUmUmUmGmGmC m |
| siANGa8-M2 | 193 | CmAmAmAmAmUmCmAmAfGfAfUmUmUmGmCmUmAmUmUmUm |
| | 194 | AmCfAmUmAS̲GfCmAmAmAmUmCmUmUfGmAfUmUmUmUmGmGmC m |
| siANGa1- | 195 | AmsAmsAmUmCmAmAfGfAfUmUmUmGmCmUmAmUmGmUm |
| M1S | 196 | AmsCfsASUmAmGfCmAmAmAmUmCmUmUfGmAfUmUmUmsUmsGm |
| siANGa1-M2S | 197 | AmsAmsAmUmCmAmAfGfAfUmUmUmGmCmUmAmUmGmUm |
| | 198 | AmsCfsAmUmASGfCmAmAmAmUmCmUmUfGmAfUmUmUmsUmsGm |
| siANGa2-M1S | 199 | AmsAmsAmUmCmAmAfGfAfUmUmUmGmCmUmAmUmCmUm |
| | 200 | AmsCfsASUmAmGfCmAmAmAmUmCmUmUfGmAfUmUmUmsUmsGm |
| siANGa2-M2S | 201 | AmsAmsAmUmCmAmAfGfAfUmUmUmGmCmUmAmUmCmUm |
| | 202 | AmsCfsAmUmASGfCmAmAmAmUmCmUmUfGmAfUmUmUmsUmsGm |
| siANGa3-M1S | 203 | AmsAmsAmUmCmAmAfGfAfUmUmUmGmCmUmAmUmAmUm |
| | 204 | AmsCfsASUmAmGfCmAmAmAmUmCmUmUfGmAfUmUmUmsUmsGm |
| siANGa3-M2S | 205 | AmsAmsAmUmCmAmAfGfAfUmUmUmGmCmUmAmUmAmUm |
| | 206 | AmsCfsAmUmASGfCmAmAmAmUmCmUmUfGmAfUmUmUmsUmsGm |
| siANGa4-M1S | 207 | AmsAmsAmUmCmAmAfGfAfUmUmUmGmCmUmAmUmUmUm |
| | 208 | AmsCfsASUmAmGfCmAmAmAmUmCmUmUfGmAfUmUmUmsUmsGm |
| siANGa4-M2S | 209 | AmsAmsAmUmCmAmAfGfAfUmUmUmGmCmUmAmUmUmUm |
| | 210 | AmsCfsAmUmASGfCmAmAmAmUmCmUmUfGmAfUmUmUmsUmsGm |
| siANGa5-M1S | 211 | CmsAmsAmAmAmUmCmAmAfGfAfUmUmUmGmCmUmAmUmGmUm |
| | 212 | AmsCfsASUmAmGfCmAmAmAmUmCmUmUfGmAfUmUmUmUmGmsGm sCm |
| siANGa5-M2S | 213 | CmsAmsAmAmAmUmCmAmAfGfAfUmUmUmGmCmUmAmUmGmUm |
| | 214 | AmsCfsAmUmASGfCmAmAmAmUmCmUmUfGmAfUmUmUmUmGmsGm sCm |
| siANGa6-M1S | 215 | CmsAmsAmAmAmUmCmAmAfGfAfUmUmUmGmCmUmAmUmCmUm |
| | 216 | AmsCfsASUmAmGfCmAmAmAmUmCmUmUfGmAfUmUmUmUmGmsGm sCm |
| siANGa6-M2S | 217 | CmsAmsAmAmAmUmCmAmAfGfAfUmUmUmGmCmUmAmUmCmUm |
| | 218 | AmsCfsAmUmASGfCmAmAmAmUmCmUmUfGmAfUmUmUmUmGmsGm sCm |
| siANGa7-M1S | 219 | CmsAmsAmAmAmUmCmAmAfGfAfUmUmUmGmCmUmAmUmAmUm |
| | 220 | AmsCfsASUmAmGfCmAmAmAmUmCmUmUfGmAfUmUmUmUmGmsGm sCm |
| siANGa7-M2S | 221 | CmsAmsAmAmAmUmCmAmAfGfAfUmUmUmGmCmUmAmUmAmUm |
| | 222 | AmsCfsAmUmASGfCmAmAmAmUmCmUmUfGmAfUmUmUmUmGmsGm sCm |
| siANGa8-M1S | 223 | CmsAmsAmAmAmUmCmAmAfGfAfUmUmUmGmCmUmAmUmUmUm |
| | 224 | AmsCfsASUmAmGfCmAmAmAmUmCmUmUfGmAfUmUmUmUmGmsGm sCm |
| siANGa8-M2S | 225 | CmsAmsAmAmAmUmCmAmAfGfAfUmUmUmGmCmUmAmUmUmUm |
| | 226 | AmsCfsAmUmASGfCmAmAmAmUmCmUmUfGmAfUmUmUmUmGmsGm sCm |
| siANGa1-M1P1 | 227 | AmAmAmUmCmAmAfGfAfUmUmUmGmCmUmAmUmGmUm |
| | 228 | P1AmCfASUmAmGfCmAmAmAmUmCmUmUfGmAfUmUmUmUmGm |
| siANGa1-M2P1 | 229 | AmAmAmUmCmAmAfGfAfUmUmUmGmCmUmAmUmGmUm |
| | 230 | P1AmCfAmUmASGfCmAmAmAmUmCmUmUfGmAtUmUmUmUmGm |
| siANGa2-M1P1 | 231 | AmAmAmUmCmAmAfGfAfUmUmUmGmCmUmAmUmCmUm |
| | 232 | P1AmCfASUmAmGfCmAmAmAmUmCmUmUfGmAtUmUmUmUmGm |
| siANGa2-M2P1 | 233 | AmAmAmUmCmAmAfGfAfUmUmUmGmCmUmAmUmCmUm |
| | 234 | P1AmCfAmUmASGfCmAmAmAmUmCmUmUfGmAfUmUmUmUmGm |
| siANGa3-M1P1 | 235 | AmAmAmUmCmAmAfGfAfUmUmUmGmCmUmAmUmAmUm |
| | 236 | P1AmCfASUmAmGfCmAmAmAmUmCmUmUfGmAfUmUmUmUmGm |
| siANGa3-M2P1 | 237 | AmAmAmUmCmAmAfGfAfUmUmUmGmCmUmAmUmAmUm |
| | 238 | P1AmCfAmUmASGfCmAmAmAmUmCmUmUfGmAfUmUmUmUmGm |
| siANGa4- | 239 | AmAmAmUmCmAmAfGfAfUmUmUmGmCmUmAmUmUmUm |
| M1P1 | 240 | P1AmCfASUmAmGfCmAmAmAmUmCmUmUfGmAfUmUmUmUmGm |
| siANGa4-M2P1 | 241 | AmAmAmUmCmAmAfGfAfUmUmUmGmCmUmAmUmUmUm |
| | 242 | P1AmCfAmUmASGfCmAmAmAmUmCmUmUfGmAfUmUmUmUmGm |
| siANGa5-M1P1 | 243 | CmAmAmAmAmUmCmAmAfGfAfUmUmUmGmCmUmAmUmGmUm |
| | 244 | P1AmCfASUmAmGfCmAmAmAmUmCmUmUfGmAfUmUmUmUmGmGm Cm |
| siANGa5-M2P1 | 245 | CmAmAmAmAmUmCmAmAfGfAfUmUmUmGmCmUmAmUmGmUm |
| | 246 | P1AmCfAmUmASGfCmAmAmAmUmCmUmUfGmAfUmUmUmUmGmGm Cm |
| siANGa6-M1P1 | 247 | CmAmAmAmAmUmCmAmAfGfAfUmUmUmGmCmUmAmUmCmUm |
| | 248 | P1AmCfASUmAmGfCmAmAmAmUmCmUmUfGmAfUmUmUmUmGmGm Cm |
| siANGa6-M2P1 | 249 | CmAmAmAmAmUmCmAmAfGfAfUmUmUmGmCmUmAmUmCmUm |
| | 250 | P1AmCfAmUmASGfCmAmAmAmUmCmUmUfGmAfUmUmUmUmGmGm Cm |
| siANGa7-M1P1 | 251 | CmAmAmAmAmUmCmAmAfGfAfUmUmUmGmCmUmAmUmAmUm |
| | 252 | P1AmCfASUmAmGfCmAmAmAmUmCmUmUfGmAfUmUmUmUmGmGm Cm |
| siANGa7-M2P1 | 253 | CmAmAmAmAmUmCmAmAfGfAfUmUmUmGmCmUmAmUmAmUm |
| | 254 | P1AmCfAmUmASGfCmAmAmAmUmCmUmUfGmAfUmUmUmUmGmGm Cm |
| siANGa8-M1P1 | 255 | CmAmAmAmAmUmCmAmAfGfAfUmUmUmGmCmUmAmUmUmUm |
| | 256 | P1AmCfASUmAmGfCmAmAmAmUmCmUmUfGmAfUmUmUmUmGmGm Cm |
| siANGa8-M2P1 | 257 | CmAmAmAmAmUmCmAmAfGfAfUmUmUmGmCmUmAmUmUmUm |
| | 258 | P1AmCfAmUmASGfCmAmAmAmUmCmUmUfGmAfUmUmUmUmGmGm Cm |
| siANGa1-M1SP1 | 259 | AmsAmsAmUmCmAmAfGfAfUmUmUmGmCmUmAmUmGmUm |
| | 260 | P1AmsCfsASUmAmGfCmAmAmAmUmCmUmUfGmAfUmUmUmsUmsGm |
| siANGa1-M2SP1 | 261 | AmsAmsAmUmCmAmAfGfAfUmUmUmGmCmUmAmUmGmUm |
| | 262 | P1AmsCfsAmUmASGfCmAmAmAmUmCmUmUfGmAfUmUmUmsUmsGm |
| siANGa2-M1SP1 | 263 | AmsAmsAmUmCmAmAfGfAfUmUmUmGmCmUmAmUmCmUm |
| | 264 | P1AmsCfsASUmAmGfCmAmAmAmUmCmUmUfGmAfUmUmUmsUmsGm |
| siANGa2-M2SP1 | 265 | AmsAmsAmUmCmAmAfGfAfUmUmUmGmCmUmAmUmCmUm |
| | 266 | P1AmsCfsAmUmASGfCmAmAmAmUmCmUmUfGmAfUmUmUmsUmsGm |
| siANGa3-M1SP1 | 267 | AmsAmsAmUmCmAmAfGfAfUmUmUmGmCmUmAmUmAmUm |
| | 268 | P1AmsCfsASUmAmGfCmAmAmAmUmCmUmUfGmAfUmUmUmsUmsGm |
| siANGa3-M2SP1 | 269 | AmsAmsAmUmCmAmAfGfAfUmUmUmGmCmUmAmUmAmUm |
| | 270 | P1AmsCfsAmUmASGfCmAmAmAmUmCmUmUfGmAfUmUmUmsUmsGm |
| siANGa4-M1SP1 | 271 | AmsAmsAmUmCmAmAfGfAfUmUmUmGmCmUmAmUmUmUm |
| | 272 | P1AmsCfsASUmAmGfCmAmAmAmUmCmUmUfGmAfUmUmUmsUmsGm |
| siANGa4-M2SP1 | 273 | AmsAmsAmUmCmAmAfGfAfUmUmUmGmCmUmAmUmUmUm |
| | 274 | P1AmsCfsAmUmASGfCmAmAmAmUmCmUmUfGmAfUmUmUmsUmsGm |
| siANGa5-M1SP1 | 275 | CmsAmsAmAmAmUmCmAmAfGfAfUmUmUmGmCmUmAmUmGmUm |
| | 276 | P1AmsCfsASUmAmGfCmAmAmAmUmCmUmUfGmAfUmUmUmUmGms GmsCm |
| siANGa5-M2SP1 | 277 | CmsAmsAmAmAmUmCmAmAfGfAfUmUmUmGmCmUmAmUmGmUm |
| | 278 | P1AmsCfsAmUmASGfCmAmAmAmUmCmUmUfGmAfUmUmUmUmGms GmsCm |
| siANGa6-M1SP1 | 279 | CmsAmsAmAmAmUmCmAmAfGfAfUmUmUmGmCmUmAmUmCmUm |
| | 280 | P1AmsCfsASUmAmGfCmAmAmAmUmCmUmUfGmAfUmUmUmUmGms GmsCm |
| siANGa6- | 281 | CmsAmsAmAmAmUmCmAmAfGfAfUmUmUmGmCmUmAmUmCmUm |
| M2SP1 | 282 | P1AmsCfsAmUmASGfCmAmAmAmUmCmUmUfGmAfUmUmUmUmGms GmsCm |
| siANGa7-M1SP1 | 283 | CmsAmsAmAmAmUmCmAmAfGfAfUmUmUmGmCmUmAmUmAmUm |
| | 284 | P1AmsCfsASUmAmGfCmAmAmAmUmCmUmUfGmAfUmUmUmUmGms GmsCm |
| siANGa7-M2SP1 | 285 | CmsAmsAmAmAmUmCmAmAfGfAfUmUmUmGmCmUmAmUmAmUm |
| | 286 | P1AmsCfsAmUmAS̲GfCmAmAmAmUmCmUmUfGmAfUmUmUmUmGms GmsCm |
| siANGa8-M1SP1 | 287 | CmsAmsAmAmAmUmCmAmAfGfAfUmUmUmGmCmUmAmUmUmUm |
| | 288 | P1AmsCfsASUmAmGfCmAmAmAmUmCmUmUfGmAfUmUmUmUmGms GmsCm |
| siANGa8-M2SP1 | 289 | CmsAmsAmAmAmUmCmAmAfGfAfUmUmUmGmCmUmAmUmUmUm |
| | 290 | P1AmsCfsAmUmAS̲GfCmAmAmAmUmCmUmUfGmAfUmUmUmUmGms GmsCm |

Wherein, capital letters C, G, U, and A indicate the base composition of the nucleotides; the lowercase m indicates that the nucleotide adjacent to the left side of the letter m is a methoxy modified nucleotide; the lowercase f indicates that the nucleotide adjacent to the left side of the letter f is a fluoro modified nucleotide; the underlined capital letter S indicates that the nucleotide adjacent to the left side of the letter S is a stably modified nucleotide; the lowercase s indicates that the two nucleotides adjacent to the left and right of the letter s are linked by phosphorothioate; and P1 represents that the nucleotide adjacent to the right side of P1 is a 5'-phosphate nucleotide or a 5'-phosphate analogue modified nucleotide. In some embodiments, S is a specific stably modified nucleotide such as moe, wherein the underlined letter combination moe indicates that the nucleotide adjacent to the left of the letter combination moe is a 2'-O-methoxyethyl modified nucleotide. In some embodiments, P1 is specifically modified VP, Ps or P, wherein the letter combination VP represents that the nucleotide adjacent to the right side of the letter combination VP is a 5'-(E)-vinylphosphonate (E-VP) modified nucleotide, the letter combination Ps represents that the nucleotide adjacent to the right side of the letter combination Ps is a phosphorothioate modified nucleotide, and the capital letter P represents that the nucleotide adjacent to the right side of the letter P is a 5'-phosphate nucleotide. In addition, each U in sequences listed in Table 3 above can be replaced by T, which will not significantly affect the activity or off-target effect of the siRNA.

In some embodiments, in each third siRNA conjugate, the conjugating group comprises a linker and a pharmaceutically acceptable targeting group and/or a delivery assisting group, and the third siRNA group, the linker, and the targeting group or the delivery assisting group are covalently or non-covalently linked in sequence. The definition and option of the targeting group are the same as those of the targeting group in the first siRNA conjugate described above. In some embodiments, the third siRNA conjugate has a structure as shown by Formula (403).

In some embodiments, the third RNAi agent comprises 2-4 third siRNAs and/or third siRNA conjugates, wherein a content of each third siRNA and/or third siRNA conjugate is equal or unequal. In some embodiments, the second RNAi agent comprises 2 third siRNAs and/or third siRNA conjugates, a ratio between them may 0.1-10, such as 0.4-5.

In some embodiments, for simple preparation, the third RNAi agent only comprises one third siRNA or third siRNA conjugate. In some embodiments, in order to obtain better delivery effect, the third RNAi agent only comprises one third siRNA conjugate.

### Solvent, pharmaceutically acceptable carrier and excipient

In some embodiments, for convenience of use, the first RNAi agent, the second RNAi agent and the third RNAi agent are present in a form of various RNAi preparations commonly used in the art.

In some embodiments, at least one or each of the first RNAi agent, the second RNAi agent and the third RNAi agent further comprises one or more of a solvent, a pharmaceutically acceptable carrier and an excipient. The following definitions and options of the solvent, the pharmaceutically acceptable carrier and the excipient are applicable to the first RNAi agent, the second RNAi agent and the third RNAi agent above.

In some embodiments, the RNAi preparation may be a liquid preparation, such as an injection solution. The liquid preparation may be an injection solution for subcutaneous injection, an injection solution for intramuscular injection or an injection solution for intravenous injection. In some embodiments, the solvent may comprise various solvents such as water, deionized water, ethanol, pH buffer and other common solvents. The pH buffer may be a trimethylolaminomethane hydrochloride buffer with a pH value of 7.5-8.5 and/or a phosphate buffer with a pH value of 5.5-8.5, for example, the pH buffer may be the phosphate buffer with the pH value of 5.5-8.5.

A dosage of the solvent is adjusted according to a required concentration of the injection solution, and on the basis of the siRNA or the siRNA group, the solvent is used to dissolve the siRNA or the siRNA to prepare 0.01 mg/mL-200 mg/mL siRNA, 20 mg/mL-200 mg/mL/, 50 mg/mL-100 mg/mL and 0.01 mg/mL-5 mg/mL siRNAs, and 0.1 mg/mL-5 mg/mL/ and 0.5 mg/mL-3 mg/mL solutions. According to the required concentration, those skilled in the art can easily determine the content of the solvent.

In order to obtain a better inhibitory effect, in some embodiments, the first RNAi agent, the second RNAi agent and the third RNAi agent are all present in a form of preparations for subcutaneous injection.

In some embodiments, in order to facilitate transportation and/or storage, all siRNAs and siRNA conjugates in the first RNAi agent and/or the second RNAi agent and/or the third RNAi agent are present in a form of powder, for example, in a form of freeze-dried powder injection, and the freeze-dried powder injection is mixed with the solvent to prepare a liquid preparation during administration.

In some embodiments, the first RNAi agent, the second RNAi agent and the third RNAi agent may also be spray preparations delivered to the lung by spraying or delivered to other organ tissues (such as the liver) through lungs by spraying, or inhalation preparations inhaled by oropharynx, or pharmaceutical preparations administered intranasally. Therefore, the first component comprises at least one of the siRNA and the siRNA conjugate and the pharmaceutically acceptable carrier and/or the excipient, and the types and contents of carriers and/or excipients in the injection solution for subcutaneous injection, the injection solution for intramuscular injection and the injection solution for intravenous injection, and in the spray preparations delivered to lungs by spraying or to other organ tissues (such as the liver) through lungs by spraying, the inhalation preparations inhaled by oropharynx or the pharmaceutical preparations administered intranasally are well-known to those skilled in the art.

The pharmaceutically acceptable carrier may be a carrier conventionally used in the field of siRNA administration, for example, but not limited to, one or more of magnetic nanoparticles (such as Fe₃O₄ or Fe₂O₃-based nanoparticle), carbon nanotubes, mesoporous silicon, calcium phosphate nanoparticles, polyethylenimine (PEI), polyamidoamine (PAMAM) dendrimer, poly(L-lysine) (PLL), chitosan, 1,2-dioleoyl-3-trimethylammonium-propane (DOTAP), poly(D&L-lactic/glycolic acid) copolymer (PLGA), poly(2-aminoethyl ethylene phosphate) (PPEEA), poly(2-dimethylaminoethyl methacrylate) (PDMAEMA), and derivatives thereof.

In the first component, the contents of the siRNA and the siRNA conjugate, and the pharmaceutically acceptable carrier are conventional contents known to those skilled in the art. In some embodiments, on the basis of the siRNA or the siRNA group, a weight ratio of the total amount of the siRNA and the siRNA conjugate to the pharmaceutically acceptable carrier may be 1: 1 to 1: 1000, 1: 1 to 1: 500, 1: 1 to 1: 200, 1: 1 to 1: 100 or 1: 1 to 1: 50.

The pharmaceutically acceptable excipient is one or more of various components commonly used in the art, such as a diluent, an adhesive, a disintegrant, a lubricant, a capsule, a protective agent, and an osmotic pressure regulator, but do not include the aforementioned solvent.

The protective agent may be at least one of inositol, sorbitol, sucrose, trehalose, mannose, maltose, lactose and glucose. The content of the protective agent may be 0.01%-30% of the total weight of the first component.

The osmotic pressure regulator may be sodium chloride and/or potassium chloride. The content of the osmotic pressure regulator enables an osmotic pressure of the injection solution to be 200-700 mOsm/kg. According to the required osmotic pressure, those skilled in the art can easily determine the content of the osmotic pressure regulator. In some embodiments, a dosage of the first component during administration will be adjusted according to different administration modes.

In some embodiments, the first siRNA, the second siRNA and/or the third siRNA are present in a form of liposomes. In some embodiments, the pharmaceutically acceptable carrier used in the liposome comprises an amine-containing transfection compound (hereinafter also referred to as an organic amine), a helper lipid and/or a pegylated lipid. The organic amine, the helper lipid and the pegylated lipid may be respectively selected from one or more of the amine-containing transfection compounds or the pharmaceutically acceptable salts or derivatives thereof, the helper lipids and the pegylated lipids as described in CN103380113A (which is incorporated herein by reference in its entirety).

In some embodiments, the organic amine may be a compound as shown by Formula (201) as described in the Chinese patent application CN103380113A or a pharmaceutically acceptable salt thereof: 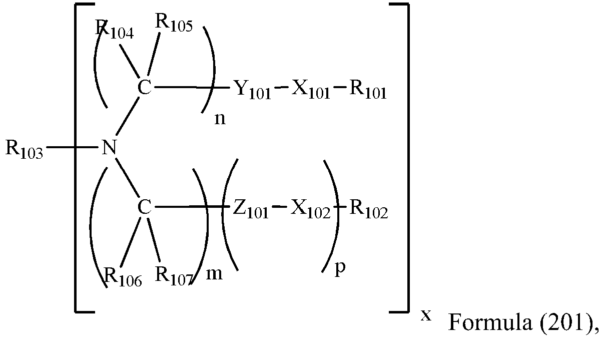 wherein:
X₁₀₁ or X₁₀₂ is independently O, S, N-A or C-A, wherein A is hydrogen or a C₁-C₂₀ hydrocarbon chain;
Y₁₀₁ or Z₁₀₁ is independently C=O, C=S, S=O, CH-OH or SO₂;
R₁₀₁, R₁₀₂, R₁₀₃, R₁₀₄, R₁₀₅, R₁₀₆ or R₁₀₇ is independently hydrogen; a cyclic or aliphatic, substituted or unsubstituted, branched or linear aliphatic group; a cyclic or aliphatic, substituted or unsubstituted, branched or linear heteroaliphatic group; a substituted or unsubstituted, branched or linear acyl group; a substituted or unsubstituted, branched or linear aryl, or a substituted or unsubstituted, branched or linear heteroaryl;
x is an integer of 1-10;
n is an integer of 1-3, m is an integer of 0-20, and p is 0 or 1, wherein if m=p=0, then R₁₀₂ is hydrogen;
and, if at least one of n or m is 2, then R₁₀₃ and the nitrogen in Formula (201) form a structure as shown by Formula (202) or (203): 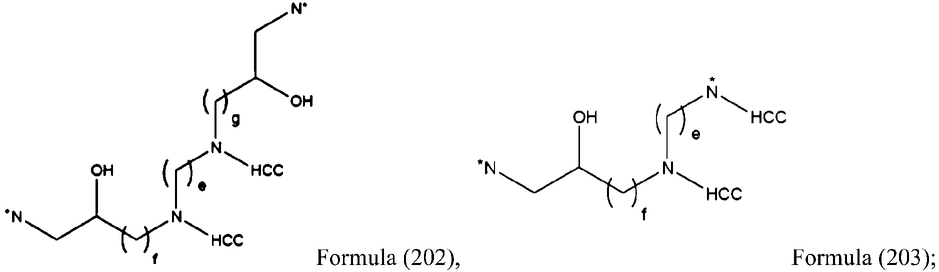
wherein g, e or f is independently an integer of 1-6, "HCC" represents a hydrocarbon chain, and each *N represents a nitrogen atom shown in Formula (201).

In some embodiments, R₁₀₃ is a polyamine. In other embodiments, R₁₀₃ is a ketal. In some embodiments, each of R₁₀₁ and R₁₀₂ in the Formula (201) is independently any substituted or unsubstituted, branched or linear alkyl or alkenyl, wherein the alkyl or alkenyl has 3 to about 20 carbon atoms, such as 8 to about 18 carbon atoms, and 0 to 4 double bonds, such as 0 to 2 double bonds.

In some embodiments, if each of n and m is independently 1-3, R₁₀₃ may be any in the following Formulae (204)-(213): 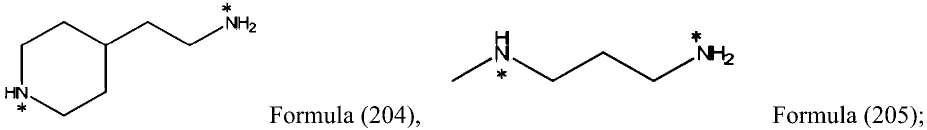 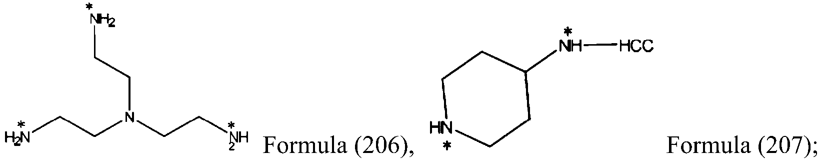 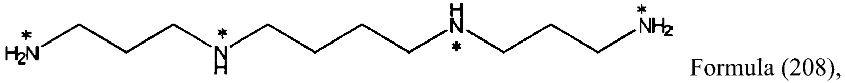 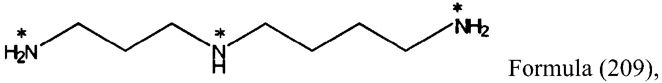 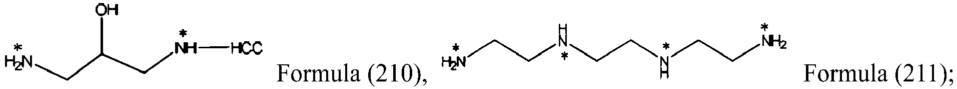 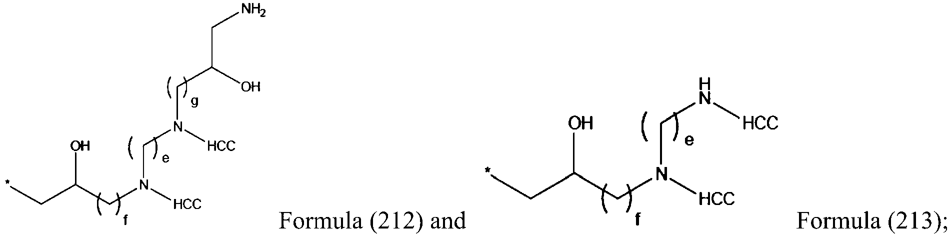 wherein, in Formula (204) to Formula (213), each of g, e and f is independently an integer of 1-6; each "HCC" represents a hydrocarbon chain, and each * represents a potential attachment point of R₁₀₃ to the nitrogen atom in Formula (201), wherein each H at any * position may be replaced to realize the attachment to the nitrogen atom in Formula (201).

Those skilled in the art can obtain the compound shown in Formula (201) by any reasonable method. In some embodiments, the compound as shown by Formula (201) may be prepared as described in the Chinese patent application CN103380113A.

In some embodiments, the organic amine may be an organic amine as shown by Formula (214) and/or an organic amine as shown by Formula (215):

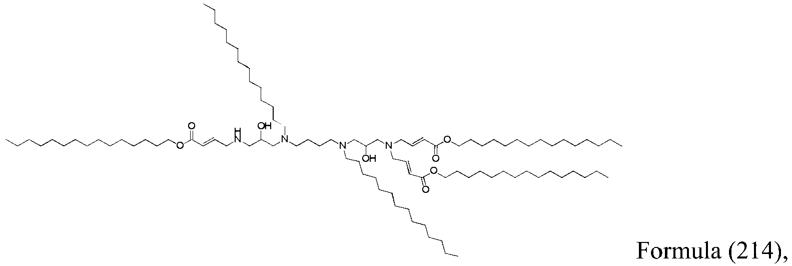

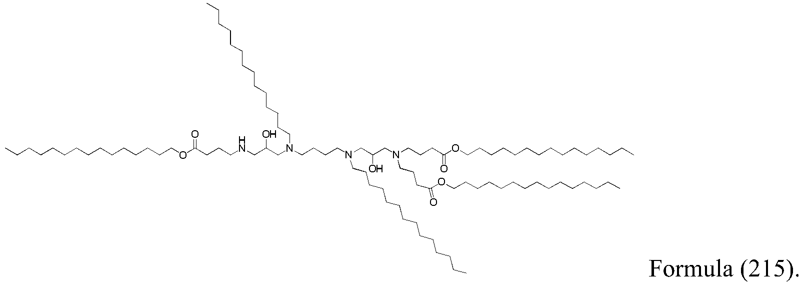

The helper lipid is a cholesterol, a cholesterol analogue and/or a cholesterol derivative.

The pegylated lipid is 1,2-dipalmitoyl-sn-glycero-3-phosphatidylethanolamine-N-[methoxy(polyethylene glycol)]-2000.

In some embodiments, the molar ratio among the organic amine, the helper lipid and the pegylated lipid in the pharmaceutical composition is (19.7-80): (19.7-80): (0.3-50); for example, the molar ratio may be (50-70): (20-40): (3-20).

In some embodiments, particles of siRNA liposomes formed by the first siRNA and/or the second siRNA and/or the third siRNA and the amine-containing transfection reagent have an average diameter from about 30 nm to about 200 nm, typically from about 40 nm to about 135 nm, and more typically, the average diameter of the liposome particles is from about 50 nm to about 120 nm, from about 50 nm to about 100 nm, from about 60 nm to about 90 nm, or from about 70 nm to about 90 nm, for example, the average diameter of the liposome particles is about 30 nm, 40 nm, 50 nm, 60 nm, 70 nm, 75 nm, 80 nm, 85 nm, 90 nm, 100 nm, 110 nm, 120 nm, 130 nm, 140 nm, 150 nm or 160 nm.

In some embodiments, in the siRNA liposome formed by the first siRNA and/or the second siRNA and/or the third siRNA and the amine-containing transfection reagent in the pharmaceutical composition of the present disclosure, the weight ratio (weight/weight ratio) of total siRNAs to total lipids (e.g., the organic amine, the helper lipid and/or the pegylated lipid), ranges from about 1: 1 to about 1: 50, from about 1: 1 to about 1: 30, from about 1: 3 to about 1: 20, from about 1: 4 to about 1: 18, from about 1: 5 to about 1: 17, from about 1: 5 to about 1: 15, from about 1: 5 to about 1: 12, from about 1: 6 to about 1: 12, or from about 1: 6 to about 1: 10. For example, the weight ratio may be about 1: 5, 1: 6, 1: 7, 1: 8, 1: 9, 1: 10, 1: 11, 1: 12, 1: 13, 1: 14, 1: 15, 1: 16, 1: 17 or 1: 18.

In some embodiments, the siRNA liposome may be prepared by various known methods, except replacing the existing siRNA with the siRNA of the present disclosure. In some embodiments, the siRNA liposome may be prepared according to the following method:
the organic amines, helper lipids and pegylated lipids are suspended in alcohol at a molar ratio as described above and mixed homogeneously to yield a lipid solution; and the alcohol is used in an amount such that the resultant lipid solution is present at a total mass concentration of 2-25 mg/mL, e.g., 8-18 mg/mL. The alcohol is a pharmaceutically acceptable alcohol, such as an alcohol that is in liquid form at about room temperature, for example, one or more of ethanol, propylene glycol, benzyl alcohol, glycerol, PEG 200, PEG 300, PEG 400, and for example, ethanol;
the siRNA is dissolved in a buffered salt solution to produce an aqueous solution of the siRNA. The buffered salt solution has a concentration of 0.05-0.5 M, such as 0.1-0.2 M. The pH of the buffered salt solution is adjusted to 4.0-5.5, such as 5.0-5.2. The buffered salt solution is used in an amount such that the siRNA is present at a concentration of less than 0.6 mg/mL, such as 0.2-0.4 mg/mL. The buffered salt is selected from one or more of soluble acetate and soluble citrate, such as sodium acetate and/or potassium acetate;
the lipid solution and the aqueous solution of the siRNA are mixed. The product obtained after mixing is incubated at a temperature of 40-60°C for at least 2 minutes (e.g., 5-30 minutes) to produce an incubated lipid formulation. The volume ratio of the lipid solution to the aqueous solution of the siRNA is 1: (2-5), such as 1: 4;
the incubated liposome formulation is concentrated or diluted, purified to remove impurities, and then sterilized to obtain the siRNA-comprising liposome, which has physicochemical parameters as follows: a pH of 6.5-8, an encapsulation percentage of more than 80%, a particle size of 40-200 nm, a polydispersity index of less than 0.30, and an osmotic pressure of 250-400 mOsm/kg; for example, the physicochemical parameters may be as follows: a pH of 7.2-7.6, an encapsulation percentage of more than 90%, a particle size of 60-100 nm, a polydispersity index of less than 0.20, and an osmotic pressure of 300-400 mOsm/kg;
the concentration or dilution step may be performed before, after or simultaneously with the step of impurity removal. The method for removing impurities may be any of various existing methods, for example, ultrafiltration under 100 KDa by using a tangential flow system and a hollow fiber column, wherein an ultrafiltration exchange solution is a phosphate buffer solution (PBS) with pH 7.4. The method for sterilization may be any of various existing methods, such as filtration sterilization on a 0.22 µm filter.

In some embodiments, the pharmaceutical composition only comprises the first RNAi agent and the second RNAi agent, and the first RNAi agent only comprises one first siRNA conjugate, or the second RNAi agent only comprises one second siRNA conjugate. Alternatively, the first RNAi agent only comprises one first siRNA conjugate, and the second RNAi agent only comprises one second siRNA conjugate.

In some embodiments, the first RNAi agent and the second RNAi agent are respectively present in a form of separate preparation for subcutaneous injection, which means that the first RNAi agent and the second RNAi agent are stored separately and may be used separately. In some embodiments, the first RNAi agent and the second RNAi agent are present in a form of a uniformly mixed preparation for subcutaneous injection.

In some embodiments, the pharmaceutical composition comprises the first RNAi agent, the second RNAi agent and the third RNAi agent, and the first RNAi agent only comprises one first siRNA conjugate, or the second RNAi agent only comprises one second siRNA conjugate, or the third RNAi agent only comprises one third siRNA conjugate. In some embodiments, the first RNAi agent only comprises one first siRNA conjugate, the second RNAi agent only comprises one second siRNA conjugate, and the third RNAi agent only comprises one third siRNA conjugate. In some embodiments, the first RNAi agent, the second RNAi agent and the third RNAi agent are all present separately. In some embodiments, one of the first RNAi agent or the second RNAi agent and the third RNAi agent are present in a form of a uniformly mixed preparation for subcutaneous injection. In some embodiments, the first RNAi agent, the second RNAi agent and the third RNAi agent are present in a form of a uniformly mixed preparation for subcutaneous injection.

### Kit

The present disclosure provides a kit for storing the pharmaceutical composition. In some embodiments, the kit disclosed herein may provide the first RNAi agent, the second RNAi agent and the third RNAi agent in the pharmaceutical composition provided by the present disclosure in one or more containers. In some embodiments, in order to use the pharmaceutical composition of the present disclosure more conveniently, the first RNAi agent and the second RNAi agent in the kit are stored separately. In some embodiments, the kit comprises one container for providing the first RNAi agent and one container for providing the second RNAi agent, and optionally, the kit further comprises one container for providing the third component. In some embodiments, the kit may further comprise other components, such as a stabilizer or a preservative.

### Use of pharmaceutical composition of the present disclosure

In another aspect, the present disclosure further discloses use of the pharmaceutical composition in preparation of a drug for treating and/or preventing a disease or symptom related to hyperlipidemia. The disease or symptom related to hyperlipidemia is selected from one or more of hypercholesterolemia, hypertriglyceridemia and familial hypercholesterolemia.

In some embodiments, the first RNAi agent and the second RNAi agent in the pharmaceutical composition are present as two separate preparations in the process of drug preparation. In some embodiments, the first RNAi agent and the second RNAi agent in the pharmaceutical composition are present as a preparation in the process of drug preparation.

### Method for treating and/or preventing disease related to hyperlipidemia

In another aspect, the present disclosure further provides a method for treating and/or preventing a disease related to hyperlipidemia, which comprises administering effective amounts of first RNAi agent and second RNAi agent to a subject in need, wherein the first RNAi agent comprises one or more first siRNAs and/or one or more first siRNA conjugates, each first siRNA is independently a siRNA capable of inhibiting *APOC3* mRNA, and each first siRNA conjugate comprises a siRNA group formed by one first siRNA and a conjugating group conjugated with the siRNA;
the second RNAi agent comprises one or more second siRNAs and/or one or more second siRNA conjugates, each second siRNA is independently a siRNA capable of inhibiting *PCSK9* mRNA, and each second siRNA conjugate comprises a siRNA group formed by one second siRNA and a conjugating group conjugated with the siRNA; and
on the basis of a total amount of siRNAs and siRNA groups in the first RNAi agent and the second RNAi agent respectively, a weight ratio of the first RNAi agent to the second RNAi agent administered to the subject is 10: 1 to 1: 10.

The disease or symptom related to hyperlipidemia is selected from one or more of hypercholesterolemia, hypertriglyceridemia and familial hypercholesterolemia.

The "effective amount" refers to a dose that can reduce a value of TC or LDL-c in a subject by at least 5%, 10% or 20% compared with the value before administration.

In some embodiments, on the basis of a total amount of siRNAs and siRNA groups in the first RNAi agent and the second RNAi agent respectively, a weight ratio of the first RNAi agent to the second RNAi agent in the administration to the subject is 100: 1 to 1: 100, such as 100: 1, 50: 1, 25: 1, 15: 1, 10: 1, 5: 1, 4: 1, 3: 1, 2: 1, 1: 1, 1: 100, 1: 50, 1: 25, 1: 15, 1: 10, 1: 5, 1: 3, 1: 4 or 1: 2. In some embodiments, preferably, the weight ratio of the first RNAi agent to the second RNAi agent in the administration to the subject is 10: 1 to 1: 10, 5: 1 to 1: 5 or 2: 1 to 1: 5. In some embodiments, in order to obtain a better treating/preventing effect, a weight of the second RNAi agent in the administration to the subject is not less than that of the first RNAi agent, and the weight ratio of the first RNAi agent to the second RNAi agent in the administration to the subject may be 1: 1 to 1: 10, 1: 1 to 1: 5 or 1: 1 to 1: 3.

In some embodiments, on the basis of the total amount of siRNAs and siRNA groups in the first RNAi agent and the second RNAi agent respectively, a dosage of the first RNAi agent administered to the subject each time is 0.5 mg/Kg-10 mg/Kg body weight, such as 0.5 mg/Kg-5 mg/Kg body weight, 0.5 mg/Kg-3 mg/Kg body weight, 1 mg/Kg-3 mg/Kg body weight, or 1 mg/Kg-5 mg/Kg body weight. A dosage of the second RNAi agent administered to the subject each time is 0.5 mg/Kg-10 mg/Kg body weight, such as 0.5 mg/Kg-9 mg/Kg body weight, 3 mg/Kg-10 mg/Kg body weight, 1 mg/Kg-9 mg/Kg body weight, 3 mg/Kg-9 mg/Kg body weight, 1 mg/Kg-6 mg/Kg body weight, or 3 mg/Kg-6 mg/Kg body weight.

In some embodiments, on the basis of the total amount of siRNAs and siRNA groups in the first RNAi agent, the dosage of the first RNAi agent administered to the subject each time is a fixed dosage of 1 mg-1000 mg, such as 1 mg-800 mg, 1 mg-600 mg, 1 mg-500 mg, 1 mg-300 mg, 1 mg-250 mg, or 1 mg-100 mg. In some embodiments, the dosage of the first RNAi agent administered to the subject is 5 mg-500 mg.

In some embodiments, on the basis of the total amount of siRNAs and siRNA groups in the second RNAi agent, the dosage of the second RNAi agent administered to the subject each time is a fixed dosage of 1 mg-1000 mg, such as 1 mg-800 mg, 1 mg-600 mg, 1 mg-500 mg, 1 mg-300 mg, 1 mg-250 mg, or 1 mg-100 mg. In some embodiments, the dosage of the second RNAi agent administered to the subject is 5 mg-800 mg.

In order to obtain a better preventing/treating effect, in some embodiments, the weight of the first RNAi agent administered to the subject is 5 mg-500 mg, and the weight of the second RNAi agent administered to the subject is 5 mg-800 mg.

In some embodiments, the method further comprises administering a third RNAi agent to the subject, wherein the third RNAi agent comprises one or more third siRNAs and/or one or more third siRNA conjugates, each third siRNA is independently a siRNA capable of inhibiting *ANGPTL3* mRNA, and each third siRNA conjugate comprises a third siRNA group formed by one third siRNA and a conjugating group conjugated with the siRNA.

In some embodiments, on the basis of a total amount of siRNAs and siRNA groups in the third RNAi agent and the second RNAi agent respectively, a weight ratio of the third RNAi agent to the second RNAi agent administered to the subject is 100: 1 to 1: 100, such as 100: 1, 50: 1, 25: 1, 15: 1, 10: 1, 5: 1, 4: 1, 3: 1, 2: 1, 1: 1, 1: 100, 1: 50, 1: 25, 1: 15, 1: 10, 1: 5, 1: 3, 1: 4 or 1: 2. In some embodiments, preferably, the weight ratio of the third RNAi agent to the second RNAi agent administered to the subject is 10: 1 to 1: 10, 5: 1 to 1: 5 or 2: 1 to 1: 5.

In some embodiments, on the basis of the total amount of siRNAs and siRNA groups in the third RNAi agent, a dosage of the third RNAi agent administered to the subject each time is a fixed dosage of 1 mg-1000 mg, such as 1 mg-800 mg, 1 mg-600 mg, 1 mg-500 mg, 1 mg-300 mg, 1 mg-250 mg, or 1 mg-100 mg.

In some embodiments, on the basis of the total amount of siRNAs and siRNA groups in the third RNAi agent, the dosage of the third RNAi agent administered to the subject each time is 0.5 mg/Kg-10 mg/Kg body weight, such as 0.5 mg/Kg-5 mg/Kg body weight, 0.5 mg/Kg-3 mg/Kg body weight, 1 mg/Kg-3 mg/Kg body weight, or 1 mg/Kg-5 mg/Kg body weight.

The definitions and options of the first RNAi agent, the second RNAi agent and the third RNAi agent are all the same as those of the first RNAi agent, the second RNAi agent and the third RNAi agent in the pharmaceutical composition of the present disclosure.

As used herein, the term "administered/administration" refers to placing the siRNA, the pharmaceutical composition and/or the siRNA conjugate into the body of the subject by a method or way of at least partially locating the siRNA, the pharmaceutical composition and/or the siRNA conjugate at a desired site to produce a desired effect. The administration routes suitable for the method of the present disclosure comprise local administration and systemic administration. In general, the local administration leads to the delivery of more siRNA, pharmaceutical composition and/or siRNA conjugate to a specific site compared with the systemic administration to the subject; while the systemic administration leads to the delivery of the siRNA, pharmaceutical composition and/or siRNA conjugate to substantially whole body of the subject. Considering that the present disclosure aims to provide a means to prevent and/or treat pathological conditions or diseases caused by the expression of specific genes in hepatocytes, and in some embodiments, it is an administration mode capable of delivering a drug to the liver.

The drug may be administered to the subject by any suitable route known in the art, comprising but not limited to oral or parenteral routes, such as intravenous administration, intramuscular administration, subcutaneous administration, transdermal administration, airway administration (aerosol), pulmonary administration, nasal administration, rectal administration and topical administration (comprising oral administration and sublingual administration).

In some embodiments, in order to obtain a better preventing/treating effect, the RNAi agents are all administered subcutaneously to the subject. For example, the first RNAi agent, the second RNAi agent and the third RNAi agent are administered in a form of a uniformly mixed subcutaneous injection solution.

In some embodiments, for separate administration, the first RNAi agent and the second RNAi agent are administered to the subject simultaneously, or the first RNAi agent and the second RNAi agent are separately administered to the subject successively within twenty four hours.

In some embodiments, for separate administration, the method further comprises administering the third RNAi agent to the subject, wherein the third RNAi agent, and the first RNAi agent and the second RNAi agent are administered to the subject simultaneously or successively within twenty four hours.

In some embodiments, a time interval of each administration among the first RNAi agent, the second RNAi agent and the third RNAi agent is 7 days to 60 weeks, 14 days to 60 weeks, 14 days to 1 year, or 14 days to 2 years. In some embodiments, in order to obtain a better treating effect, the time interval of each administration among the first RNAi agent, the second RNAi agent and the third RNAi agent is 14 days to 1 year, 3 weeks to 1 year, 1 month to 1 year, 2 months to 1 year, 3 months to 1 year, or 6 months to 1 year. Specifically, in some embodiments, the time interval of each administration among the first RNAi agent, the second RNAi agent and the third RNAi agent is 14 days, 21 days, 1 month, 2 months, 3 months, 6 months, 1 year, or 2 years.

Hereinafter, the present disclosure will be further described by examples, but is not limited thereto in any respect.

### EXAMPLES

Unless otherwise specified, the agents and culture media used in following examples are all commercially available, and the procedures used such as nucleic acid electrophoresis and real-time PCR are all performed according to methods described in Molecular Cloning (Cold Spring Harbor Laboratory Press (1989)).

### Preparation Example 1 Preparation of pharmaceutical composition provided by the present disclosure

### 1-1 Preparation of first siRNA conjugate and second siRNA conjugate in pharmaceutical composition of the present disclosure

According to the preparation method described in Preparation Example 13 of CN110959011A, a first siRNA conjugate and a second siRNA conjugate were prepared in a form of freeze-dried powder, the only difference was that sense strands and anti-sense strands in the first siRNA conjugate and the second siRNA conjugate had sequences shown in Table 4 below, and the sense strand and the anti-sense strand were synthesized respectively. A conjugate 1 and a conjugate 2 were respectively diluted to a concentration of 0.2 mg/mL (on the basis of the amount of siRNA groups) with ultra-pure water (Milli-Q ultra-pure water meter, electrical resistivity 18.2 MΩ*cm (25°C)), and then subjected to molecular weight detection by Liquid Chromatography-Mass Spectrometry (LC-MS, purchased from Waters Company, model: LCT Premier). A theoretical value of a molecular weight (MW) of the sense strand of the first siRNA conjugate was 7581.42 and a measured value was 7581.22, and a theoretical value of a molecular weight of the anti-sense strand was 6948.55 and a measured value was 6948.05. A theoretical value of a molecular weight of the sense strand of the second siRNA conjugate was 7426.26 and a measured value was 7425.55, and a theoretical value of a molecular weight of the anti-sense strand was 7084.88 and a measured value was 7084.14. The measured values were consistent with the theoretical values, indicating that the first and second siRNA conjugates synthesized were double-stranded nucleic acid sequences in object design. The first siRNA conjugate and the second siRNA conjugate obtained had a structure as shown by Formula (403):

**Table 4 Sequences of sense strands and anti-sense strands in siRNA conjugates**

| No. of preparation example | Direction from 5' to 3' of sequence | | SEQ ID NO |
|---|---|---|---|
| First siRNA conjugate | Sense strand | UmsUmsAmAmAmAmGfGfGfAmCmAmGm UmAmUmUmCmUm | 53 |
| | Anti-sen se strand | AmsGfsAmoeAmUmAfCmUmGmUmCmCmC mUfUmUfUmAmAmsUmsUm | 291 |
| Second siRNA conjugate | Sense strand | CmsUmsGmUmUmUmUfGfCfUmUmUmUm GmUmAmAmCmUm | 123 |
| | Anti-sen se strand | AmsGfsUmUmAmCfAmAmAmAmGmCmAm AfAmAfCmAmGmsGmsUm | 124 |

wherein, capital letters C, G, U, A and T indicate the base composition of the nucleotides; the lowercase m indicates that the nucleotide adjacent to the left side of the letter m is a methoxy modified nucleotide; the lowercase f indicates that the nucleotide adjacent to the left side of the letter f is a fluoro modified nucleotide; the underlined letter combination moe indicates that the nucleotide adjacent to the left of the letter combination moe is a ribose 2'-O-methoxyethyl modified nucleotide; the lowercase s indicates that the two nucleotides adjacent to the left and right of the letter s are linked by phosphorothioate; and P represents that the nucleotide adjacent to the right side of the letter P is a 5'-phosphate nucleotide.

### 1-2 Preparation of first RNAi agent and second RNAi agent in pharmaceutical composition of the present disclosure

The first siRNA conjugate powder prepared in 1-1 was dissolved into solutions at concentrations of 0.2 mg/mL and 0.6 mg/mL respectively with PBS, and the solutions were respectively recorded as a low-concentration first RNAi agent and a high-concentration first RNAi agent.

The second siRNA conjugate powder prepared in 1-1 was dissolved into solutions at concentrations of 0.6 mg/mL and 1.2 mg/mL respectively with PBS, and the solutions were respectively recorded as a low-concentration second RNAi agent and a high-concentration second RNAi agent.

### 1-3 Preparation of pharmaceutical composition of the present disclosure

0.9 mL of low-concentration first RNAi agent and 0.9 mL of low-concentration second RNAi agent were uniformly mixed to prepare a pharmaceutical composition I, wherein a weight ratio of the first RNAi agent to the second RNAi agent was 1: 3.

0.9 mL of high-concentration first RNAi agent and 0.9 mL of low-concentration second RNAi agent were uniformly mixed to prepare a pharmaceutical composition II, wherein a weight ratio of the first RNAi agent to the second RNAi agent was 1: 1.

0.9 mL of high-concentration first RNAi agent and 0.9 mL of high-concentration second RNAi agent were uniformly mixed to prepare a pharmaceutical composition III, wherein a weight ratio of the first RNAi agent to the second RNAi agent was 1: 2.

### Experimental Example 1 Active effect of pharmaceutical composition provided by the disclosure in mice

35 female *hAPOC3* transgenic mice (species: (C57BL/6J×CBA/J)F1, grade: SPF, purchased from SPF Company were randomly divided into 7 groups, with 5 mice in each group, numbered as Group 1-Group 7 respectively, and this kind of mice had severe hypertriglyceridemia and significantly increased cholesterol.

The mice in Group 1-Group 7 were administered with the agents on the day of grouping according to administration schemes in Table 5 below, and the administration day was recorded as the first day.

**Table 5. Administration schemes for mice in Group 1-Group 7**

| Group | Administration scheme | First RNAi agent Dosage (mg/Kg) | Second RNAi agent Dosage (mg/Kg) |
|---|---|---|---|
| 1 | Subcutaneous injection of low-concentration first RNAi at dosage of 5 mL/Kg | 1 | 0 |
| 2 | Subcutaneous injection of high-concentration first RNAi at dosage of 5 mL/Kg | 3 | 0 |
| 3 | Subcutaneous injection of low-concentration second RNAi at dosage of 5 mL/Kg | 0 | 3 |
| 4 | Subcutaneous injection of high-concentration second RNAi at dosage of 5 mL/Kg | 0 | 6 |
| 5 | Subcutaneous injection of pharmaceutical composition I at dosage of 5 mL/Kg | 1 | 3 |
| 6 | Subcutaneous injection of pharmaceutical composition II at dosage of 5 mL/Kg | 3 | 3 |
| 7 | Subcutaneous injection of pharmaceutical composition III at dosage of 5 mL/Kg | 3 | 6 |

Blood was taken from the anterior orbit of all mice on a first day before administration (D1), an eighth day (D8) and a fifteenth day (D15), the mice were fasted for 4-6 hours before taking blood, and 0.2 mL of blood was taken from the orbit of the mice after anesthetization with 70% CO₂ by an EDTAK₂ anti-coagulation blood collection tube. The obtained blood was centrifuged at 2-8°C for 15 minutes with 1800 g centrifugal force, then a supernatant was taken to separate plasma, and the plasma was stored in a refrigerator at -80°C.

The obtained plasma sample was sent to Labcorp Pharmaceutical Research & Development (Shanghai) Co., Ltd. for determination of contents of TC and LDL-c in the plasma, wherein, normalized values of TC and LDL-c in plasma = (contents after administration (D8 or D15) / contents before administration (D1))*100%. The results of various groups were summarized in Table 6 below.

**Table 6 Normalized values of contents of TC and LDL-C in mice in groups 1-7**

| Group | TC (%) | | LDL-C (%) | | Corresponding administration dosage |
|---|---|---|---|---|---|
| | D8 | D15 | D8 | D15 | |
| 1 | 72.3 | 70.9 | 47.6 | 49.0 | First RNAi agent 1 mg/kg |
| 2 | 66.0 | 59.7 | 41.4 | 40.2 | First RNAi agent 3 mg/kg |
| 3 | 92.8 | 95.2 | 54.7 | 57.5 | Second RNAi agent 3 mg/kg |
| 4 | 100.7 | 95.3 | 58.6 | 56.7 | Second RNAi agent 6 mg/kg |
| 5 | 63.0 | 62.8 | 33.0 | 36.7 | Pharmaceutical composition I 4 mg/kg |
| 6 | 54.4 | 53.9 | 27.2 | 31.4 | Pharmaceutical composition II 6 mg/kg |
| 7 | 49.2 | 47.8 | 22.1 | 24.3 | Pharmaceutical composition III 9 mg/kg |

It can be seen from the retults in Table 6 that, for a change of the level of TC, on one hand, under the condition that the total administration dosages are all 6 mg/Kg, mice of group 4 separately administered with 6 mg/Kg RNAi agent for inhibiting the *PCSK9* mRNA show almost no reduction of TC *in vivo,* while mice in the group 6 administered with the pharmaceutical composition of the present disclosure (3 mg/Kg RNAi agent for inhibiting the *APOC3* mRNA and 3 mg/Kg RNAi agent for inhibiting the *PCSK9* mRNA) show more than 45% reduction rate of TC *in vivo* (reduction rate of TC = 100% - normalized value of content of TC). Even if reduction rates of TC in mice in the group 2 and the group 3 administered with the RNAi agent for inhibiting the *APOC3* mRNA and the RNAi agent for inhibiting the *PCSK9* mRNA respectively are added, a sum of the reduction rates of TC is also lower than 45%. Under the condition that the total administration dosages are all 4 mg/Kg, when reduction rates of TC in mice in the group 1 and the group 3 administered with 1 mg/Kg RNAi agent for inhibiting the *APOC3* mRNA and 3 mg/Kg RNAi agent for inhibiting the *PCSK9* mRNA respectively are added, a maximum sum of the reduction rates of TC is 34.9%, while a reduction rate of TC in mice in the group 5 administered with the pharmaceutical composition of the present disclosure (1 mg/Kg RNAi agent for inhibiting the *APOC3* mRNA and 3 mg/Kg RNAi agent for inhibiting the *PCSK9* mRNA) can reach 37.2%. On the other hand, taking a change of a level of TC on an 8^{th} day after administration as an example, under the condition that the total administration dosages are all 6 mg/Kg, mice in the group 4 separately administered with 6 mg/Kg RNAi agent for inhibiting the *PCSK9* mRNA show no reduction of TC *in vivo,* while mice in the group 6 administered with the pharmaceutical composition of the present disclosure (3 mg/Kg RNAi agent for inhibiting the *APOC3* mRNA and 3 mg/Kg RNAi agent for inhibiting the *PCSK9* mRNA) show 45.6% reduction rate of TC. Even if reduction rates of TC in mice in the group 2 and the group 3 administered with the RNAi agent for inhibiting the *APOC3* mRNA (3 mg/Kg) and the RNAi agent for inhibiting the *PCSK9* mRNA (3mg/Kg) respectively are added, a sum of the reduction rates of TC is only 33.3%. Under the condition that the total administration dosages are all 4 mg/Kg, when reduction rates of TC in mice in the group 1 and the group 3 administered with 1 mg/Kg RNAi agent for inhibiting the *APOC3* mRNA and 3 mg/Kg RNAi agent for inhibiting the *PCSK9* mRNA respectively are added, a maximum sum of the reduction rates of TC is 34.9%, while a reduction rate of TC in mice in the group 5 administered with the pharmaceutical composition of the present disclosure (1 mg/Kg RNAi agent for inhibiting the *APOC3* mRNA and 3 mg/Kg RNAi agent for inhibiting the *PCSK9* mRNA) can reach 37.0%.

For a change of the level of LDL-c, on one hand, under the condition that the total administration dosages are all 6 mg/Kg, mice in the group 4 separately administered with the RNAi agent for inhibiting the *PCSK9* mRNA show 43.3% maximum reduction rate of LDL-c *in vivo* (reduction rate of LDL-c = 100% - normalized value of content of LDL-c), while mice in the group 6 administered with the pharmaceutical composition of the present disclosure show a reduction rate of LDL-c as high as 72.8% *in vivo.* Moreover, the level of LDL-c in plasma cannot be reduced to below 50% regardless of the separate administration of the high-dose RNAi agent for inhibiting the *APOC3* mRNA or the separate administration of the high-dose RNAi agent for inhibiting the *PCSK9* mRNA, but the mice in the group 5 administered with the low-dose (4 mg/kg) pharmaceutical composition of the present disclosure have already shown a reduction rate of LDL-c as high as 67% *in vivo.* Taking a change of a level of LDL-c on an 8^{th} day after administration as an example, under the condition that the total administration dosages are all 6 mg/Kg, mice in the group 4 separately administered with the RNAi agent for inhibiting the *PCSK9* mRNA show 41.4% reduction rate of LDL-c, while mice in the group 6 administered with the pharmaceutical composition of the present disclosure (3 mg/Kg RNAi agent for inhibiting the *APOC3* mRNA and 3 mg/Kg RNAi agent for inhibiting the *PCSK9* mRNA) show a reduction rate of LDL-c as high as 72.8%.

The above results show that, compared with separate first RNAi agent or second RNAi agent, the composition and the treatment method provided by the present disclosure both have a significantly better effect on reducing the level of TC and the level of LDL-c, show a significant synergistic effect, especially on reducing the level of LDL-c which has attracted much attention, and unexpectedly break through a limitation on reducing the level of LDL-c by separate administration of the first RNAi agent or the second RNAi agent. The pharmaceutical composition and the treatment method of the present disclosure have a good application prospect in preventing and/or treating a symptom or disease related to hyperlipidemia.

Some embodiments of the present disclosure are described in detail above, but the present disclosure is not limited to the specific details in the above embodiments. Within the technical concept of the present disclosure, the technical solutions of the present disclosure may have many simple modifications, and these simple modifications all belong to the scope of protection of the present disclosure.

In addition, it should be noted that the specific technical features described in the above embodiments can be combined in any suitable way without contradiction. In order to avoid unnecessary repetition, various possible combinations are not explained separately in the present disclosure.

In addition, different embodiments of the present disclosure can be combined at will, as long as the combination does not violate the idea of the present disclosure, and the combination should also be regarded as the content disclosed by the present disclosure.

## Claims

1. A pharmaceutical composition, comprising a first RNAi agent and a second RNAi agent, wherein the first RNAi agent comprises one or more first siRNAs and/or one or more first siRNA conjugates, each first siRNA is independently a siRNA capable of inhibiting *APOC3* mRNA, and each first siRNA conjugate comprises a siRNA group formed by one first siRNA and a conjugating group conjugated with the siRNA;
the second RNAi agent comprises one or more second siRNAs and/or one or more second siRNA conjugates, each second siRNA is independently a siRNA capable of inhibiting *PCSK9* mRNA, and each second siRNA conjugate comprises a siRNA group formed by one second siRNA and a conjugating group conjugated with the siRNA; and
on the basis of a total amount of siRNAs and siRNA groups in the first RNAi agent and the second RNAi agent respectively, a weight ratio of the first RNAi agent to the second RNAi agent in the pharmaceutical composition is 10: 1 to 1: 10.

2. The pharmaceutical composition according to claim 1, wherein the weight ratio of the first RNAi agent to the second RNAi agent in the pharmaceutical composition is 5: 1 to 1: 5.

3. The pharmaceutical composition according to claim 2, wherein the weight ratio of the first RNAi agent to the second RNAi agent in the pharmaceutical composition is 2: 1 to 1: 5.

4. The pharmaceutical composition according to claim 1, wherein, on the basis of the total amount of siRNAs and siRNA groups in the first RNAi agent and the second RNAi agent respectively, in each pharmaceutical composition, a content of the first RNAi agent is 5 mg-500 mg, and a content of the second RNAi agent is 5 mg-800 mg.

5. The pharmaceutical composition according to claim 4, wherein in each pharmaceutical composition, the content of the first RNAi agent is 25 mg-250 mg, and the content of the second RNAi agent is 50 mg-500 mg.

6. The pharmaceutical composition according to claim 1, wherein the first RNAi agent and/or the second RNAi agent further comprises one or more of a solvent, a pharmaceutically acceptable carrier and an excipient.

7. The pharmaceutical composition according to claim 1, wherein each first siRNA comprises a sense strand and an anti-sense strand, and each nucleotide in the sense strand and the anti-sense strand is independently a modified or unmodified nucleotide; and a length of the sense strand is 18-23 nucleotides, a length of the anti-sense strand is 18-26 nucleotides, the sense strand and the anti-sense strand are basically or completely complementary in reverse, the first siRNA comprises at least one siRNA, and the sense strand of the first siRNA has at least 15 nucleotides continuously consistent with those in a nucleotide sequence shown in SEQ ID NO: 1, or the sense strand has at least 15 nucleotides continuously consistent with those in a nucleotide sequence shown in SEQ ID NO: 2:
5'-UGGCCUCCCAAUAAAGCUGGACAAGAAGCU-3' (SEQ ID NO: 1);
5'-UGCUUAAAAGGGACAGUAUUCUCAGUGCUC-3' (SEQ ID NO: 2).

8. The pharmaceutical composition according to claim 7, wherein all nucleotides in the sense strand and the anti-sense strand are modified nucleotides; and according to a direction from a 5' terminal to a 3' terminal, 7^{th} to 9^{th} nucleotides of the sense strand are 2'-fluoro modified nucleotides, according to the direction from the 5' terminal to the 3' terminal, 2^{nd}, 6^{th}, 14^{th} and 16^{th} nucleotides of the anti-sense strand are 2'-fluoro modified nucleotides, and nucleotides in other positions of the sense strand and the anti-sense strand are independently one of non-fluoro modified nucleotides.

9. The pharmaceutical composition according to claim 8, wherein the first siRNA has a sequence shown as siAPOC3b1-M1SP1 or siAPOC3b3-M1S.

10. The pharmaceutical composition according to claim 1, wherein each second siRNA comprises a sense strand and an anti-sense strand, and each nucleotide in the sense strand and the anti-sense strand is independently a modified or unmodified nucleotide; and a length of the sense strand is 18-23 nucleotides, a length of the anti-sense strand is 18-26 nucleotides, the second siRNA comprises at least one siRNA, the sense strand and the anti-sense strand of the siRNA are basically or completely complementary in reverse, and the sense strand of the second siRNA has at least 15 nucleotides continuously consistent with those in a nucleotide sequence shown in SEQ ID NO: 3:
5'-UUCUAGACCUGUUUUGCUUUUGUAACUUGA-3' (SEQ ID NO: 3).

11. The pharmaceutical composition according to claim 10, wherein all nucleotides in the sense strand and the anti-sense strand are modified nucleotides; and according to a direction from a 5' terminal to a 3' terminal, 7^{th} to 9^{th} nucleotides of the sense strand are 2'-fluoro modified nucleotides, according to the direction from the 5' terminal to the 3' terminal, 2^{nd} 6^{th}, 14^{th} and 16^{th} nucleotides of the anti-sense strand are 2'-fluoro modified nucleotides, and nucleotides in other positions of the sense strand and the anti-sense strand are independently one of non-fluoro modified nucleotides.

12. The pharmaceutical composition according to claim 11, wherein the second siRNA has a sequence shown as siPCSKd1-M1S.

13. The pharmaceutical composition according to claim 1, wherein, in each first siRNA conjugate and second siRNA conjugate, the conjugating group comprises a linker and a pharmaceutically acceptable targeting group, and the first siRNA group or the second siRNA group, the linker and the targeting group are covalently or non-covalently linked in sequence, and each targeting group is selected from ligands capable of binding with ASGPR on a cell surface.

14. The pharmaceutical composition according to claim 13, wherein the first siRNA conjugate and/or the second siRNA conjugate has a structure as shown by Formula (403): 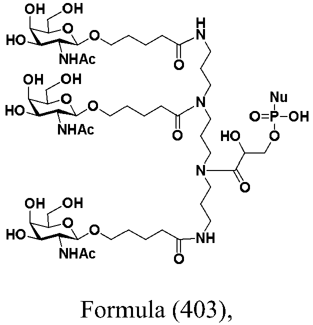 wherein, Nu represents the first siRNA group or the second siRNA group.

15. The pharmaceutical composition according to claim 1, wherein the first RNAi agent only comprises one first siRNA conjugate and/or the second RNAi agent only comprises one second siRNA conjugate; the sense strand and the anti-sense strand of the first siRNA are basically or completely complementary in reverse, and the sense strand of the first siRNA has at least 15 nucleotides continuously consistent with those in the nucleotide sequence shown in SEQ ID NO: 1, or the sense strand of the first siRNA has at least 15 nucleotides continuously consistent with those in the nucleotide sequence shown in SEQ ID NO: 2; and the sense strand and the anti-sense strand of the second siRNA are basically or completely complementary in reverse, and the sense strand of the second siRNA has at least 15 nucleotides continuously consistent with those in the nucleotide sequence shown in SEQ ID NO: 3;
5'-UGGCCUCCCAAUAAAGCUGGACAAGAAGCU-3' (SEQ ID NO: 1);
5'-UGCUUAAAAGGGACAGUAUUCUCAGUGCUC-3' (SEQ ID NO: 2);
5'-UUCUAGACCUGUUUUGCUUUUGUAACUUGA-3' (SEQ ID NO: 3).

16. The pharmaceutical composition according to claim 15, wherein the first RNAi agent only comprises one first siRNA conjugate, and the second RNAi agent only comprises one second siRNA conjugate.

17. The pharmaceutical composition according to claim 1, wherein the first RNAi agent and the second RNAi agent are respectively present in a form of separate preparation for subcutaneous injection; or, the first RNAi agent and the second RNAi agent are present in a form of a uniformly mixed preparation for subcutaneous injection.

18. The pharmaceutical composition according to any one of claims 1 to 17, wherein the pharmaceutical composition further comprises a third RNAi agent, the third RNAi agent comprises one or more third siRNAs and/or one or more third siRNA conjugates, each third siRNA is independently a siRNA capable of inhibiting *ANGPTL3* mRNA, and each third siRNA conjugate comprises a third siRNA group formed by one third siRNA and a conjugating group conjugated with the siRNA.

19. The pharmaceutical composition according to claim 18, wherein on the basis of a total amount of siRNAs and siRNA groups in the third RNAi agent and the second RNAi agent respectively, a weight ratio of the third RNAi agent to the second RNAi agent in the pharmaceutical composition is 10: 1 to 1: 10.

20. The pharmaceutical composition according to claim 18 or 19, wherein each third siRNA comprises a sense strand and an anti-sense strand, and each nucleotide in the sense strand and the anti-sense strand is independently a modified or unmodified nucleotide; and a length of the sense strand is 18-23 nucleotides, a length of the anti-sense strand is 18-26 nucleotides, the third siRNA comprises at least one siRNA, the sense strand and the anti-sense strand of the siRNA are basically or completely complementary in reverse, and the sense strand of the third siRNA has at least 17 nucleotides continuously consistent with those in a nucleotide sequence shown in SEQ ID NO: 4:
5'-AGCCAAAAUCAAGAUUUGCUAUGUUAGACG-3' (SEQ ID NO: 4).

21. The pharmaceutical composition according to claim 20, wherein the third siRNA has a sequence shown as siANGa1-M1S or siANGa1-M2S.

22. Use of the pharmaceutical composition according to any one of claims 1 to 21 in preparation of a drug for treating and/or preventing a disease or symptom related to hyperlipidemia.

23. The use according to claim 22, wherein the disease or symptom related to hyperlipidemia is selected from one or more of hypercholesterolemia, hypertriglyceridemia, mixed hyperlipidemia and familial hypercholesterolemia.

24. A method for treating and/or perventing a disease or symptom related to hyperlipidemia, comprising administering effective amounts of a first RNAi agent and a second RNAi agent to a subject in need, wherein the first RNAi agent comprises one or more first siRNAs and/or one or more first siRNA conjugates, each first siRNA is independently a siRNA capable of inhibiting *APOC3* mRNA, and each first siRNA conjugate comprises a siRNA group formed by one first siRNA and a conjugating group conjugated with the siRNA;
the second RNAi agent comprises one or more second siRNAs and/or one or more second siRNA conjugates, each second siRNA is independently a siRNA capable of inhibiting *PCSK9* mRNA, and each second siRNA conjugate comprises a siRNA group formed by one second siRNA and a conjugating group conjugated with the siRNA; and
on the basis of a total amount of siRNAs and siRNA groups in the first RNAi agent and the second RNAi agent respectively, a weight ratio of the first RNAi agent to the second RNAi agent administered to the subject is 10: 1 to 1: 10.

25. The method according to claim 24, wherein the weight ratio of the first RNAi agent to the second RNAi agent administered to the subject is 5: 1 to 1: 5.

26. The method according to claim 25, wherein the weight ratio of the first RNAi agent to the second RNAi agent administered to the subject is 2: 1 to 1: 5.

27. The method according to claim 24, wherein, on the basis of the total amount of siRNAs and siRNA groups, a dosage of the first RNAi agent administered to the subject each time is 0.5 mg/Kg-5 mg/Kg body weight of the subject, and a dosage of the second RNAi agent administered to the subject each time is 0.5 mg/Kg-9 mg/Kg body weight of the subject.

28. The method according to claim 27, wherein the dosage of the first RNAi agent administered to the subject each time is 1 mg/Kg-3 mg/Kg body weight of the subject, and the dosage of the second RNAi agent administered to the subject each time is 3 mg/Kg- 9 mg/Kg body weight of the subject.

29. The method according to claim 24, wherein the dosage of the first RNAi agent administered to the subject each time is a fixed dosage of 5 mg-500 mg, and the dosage of the second RNAi agent administered to the subject each time is a fixed dosage of 5 mg-800 mg.

30. The method according to claim 29, wherein the dosage of the first RNAi agent administered to the subject each time is a fixed dosage of 25 mg-250 mg, and the dosage of the second RNAi agent administered to the subject each time is a fixed dosage of 50 mg-500 mg.

31. The method according to claim 24, wherein each nucleotide in a sense strand and an anti-sense strand is independently a modified or unmodified nucleotide; and a length of the sense strand is 18-23 nucleotides, a length of the anti-sense strand is 18-26 nucleotides, the sense strand and the anti-sense strand are basically or completely complementary in reverse, the first siRNA comprises at least one siRNA, and the sense strand of the first siRNA has at least 15 nucleotides continuously consistent with those in a nucleotide sequence shown in SEQ ID NO: 1, or the sense strand has at least 15 nucleotides continuously consistent with those in a nucleotide sequence shown in SEQ ID NO: 2:
5'-UGGCCUCCCAAUAAAGCUGGACAAGAAGCU-3' (SEQ ID NO: 1);
5'-UGCUUAAAAGGGACAGUAUUCUCAGUGCUC-3' (SEQ ID NO: 2).

32. The method according to claim 31, wherein all nucleotides in the sense strand and the anti-sense strand are modified nucleotides; and according to a direction from a 5' terminal to a 3' terminal, 7^{th} to 9^{th} nucleotides of the sense strand are 2'-fluoro modified nucleotides, according to the direction from the 5' terminal to the 3' terminal, 2^{nd}, 6^{th}, 14^{th} and 16^{th} nucleotides of the anti-sense strand are 2'-fluoro modified nucleotides, and other nucleotides of the anti-sense strand are independently one of non-fluoro modified nucleotides.

33. The method according to claim 32, wherein the first siRNA has a sequence shown as siAPOC3b1-M1SP1 or siAPOC3b3-M1S.

34. The method according to claim 24, wherein each second siRNA comprises a sense strand and an anti-sense strand, and each nucleotide in the sense strand and the anti-sense strand is independently a modified or unmodified nucleotide; and a length of the sense strand is 18-23 nucleotides, a length of the anti-sense strand is 18-26 nucleotides, the sense strand and the anti-sense strand are basically or completely complementary in reverse, the second siRNA comprises at least one siRNA, and the sense strand of the second siRNA has at least 15 nucleotides continuously consistent with those in a nucleotide sequence shown in SEQ ID NO: 3:
5'-UUCUAGACCUGUUUUGCUUUUGUAACUUGA-3' (SEQ ID NO: 3).

35. The method according to claim 34, wherein all nucleotides in the sense strand and the anti-sense strand are modified nucleotides; and according to a direction from a 5' terminal to a 3' terminal, 7^{th} to 9^{th} nucleotides of the sense strand are 2'-fluoro modified nucleotides, according to the direction from the 5' terminal to the 3' terminal, 2^{nd}, 6^{th}, 14^{th} and 16^{th} nucleotides of the anti-sense strand are 2'-fluoro modified nucleotides, and nucleotides in other positions of the sense strand and the anti-sense strand are independently one of non-fluoro modified nucleotides.

36. The method according to claim 35, wherein the second siRNA has a sequence shown as siPCSKd1-M1S.

37. The method according to claim 24, wherein the method further comprises administering a third RNAi agent to the subject, the third RNAi agent comprises one or more third siRNAs and/or one or more third siRNA conjugates, each third siRNA is independently a siRNA capable of inhibiting *ANGPTL3* mRNA, and each third siRNA conjugate comprises a third siRNA group formed by one third siRNA and a conjugating group conjugated with the siRNA.

38. The method according to claim 37, wherein on the basis of a total amount of siRNAs and siRNA groups in the third RNAi agent and the second RNAi agent respectively, a weight ratio of the third RNAi agent to the second RNAi agent administered to the subject each time is 10: 1 to 1: 10.

39. The method according to claim 37, wherein each third siRNA comprises a sense strand and an anti-sense strand, and each nucleotide in the sense strand and the anti-sense strand is independently a modified or unmodified nucleotide; and a length of the sense strand is 18-23 nucleotides, a length of the anti-sense strand is 18-26 nucleotides, the sense strand and the anti-sense strand are basically or completely complementary in reverse, the third siRNA comprises at least one siRNA, and the sense strand of the third siRNA has at least 15 nucleotides continuously consistent with those in a nucleotide sequence shown in SEQ ID NO: 4:
5'-AGCCAAAAUCAAGAUUUGCUAUGUUAGACG-3' (SEQ ID NO: 4).

40. The method according to claim 39, wherein the third siRNA has a sequence shown as siANGa1-M1S or siANGa1-M2S.

41. The method according to claim 24, wherein the first RNAi agent and the second RNAi agent are administered to the subject simultaneously, or the first RNAi agent and the second RNAi agent are separately administered to the subject successively within twenty four hours.

42. The method according to claim 37, wherein the method further comprises administering an effective amount of the third RNAi agent to the subject, wherein the third RNAi agent, the first RNAi agent and the second RNAi agent are administered to the subject simultaneously or successively within twenty four hours.

43. The method according to claim 24 or 37, wherein all the RNAi agents are administered subcutaneously to the subject.

44. The method according to any one of claims 24 to 43, wherein the disease or symptom is selected from one or more of hypercholesterolemia, hypertriglyceridemia, mixed hyperlipidemia and familial hypercholesterolemia.
